# EUROPÄISCHE PATENTSCHRIFT

(11) **EP 1 940 763 B1**
(45) Veröffentlichungstag und Bekanntmachung des Hinweises auf die Patenterteilung: **07.04.2010**
(21) Anmeldenummer: 06806997.0
(22) Anmeldetag: 05.10.2006
(51) Int. Cl.: C07C 45/33, C07C 45/35, C07C 51/215, C07C 51/25, C07C 47/22, C07C 57/04

(54) **VERFAHREN ZUR HERSTELLUNG VON ACROLEIN, ODER ACRYLSÄURE ODER DEREN GEMISCH AUS PROPAN**
PROCESS FOR PREPARING ACROLEIN OR ACRYLIC ACID OR A MIXTURE THEREOF FROM PROPANE
PROCEDE POUR PREPARER DE L'ACROLEINE OU DE L'ACIDE ACRYLIQUE OU LEUR MELANGE, A PARTIR DE PROPANE

(30) Priorität: 14.10.2005 DE 102005049699; 14.10.2005 US 726176 P; 25.10.2005 DE 102005051401; 25.10.2005 US 729750 P; 03.11.2005 DE 102005052917; 03.11.2005 US 732657 P
(43) Veröffentlichungstag der Anmeldung: 09.07.2008
(73) Patentinhaber: BASF SE, 67056 Ludwigshafen (DE)
(72) Erfinder: KLANNER, Catharina, 68163 Mannheim (DE); DIETERLE, Martin, 68167 Mannheim (DE); SCHINDLER, Götz-Peter, 67071 Ludwigshafen (DE); CHENG, Tsung-Chieh, 64646 Heppenheim (DE); MÜLLER-ENGEL, Klaus Joachim, 76297 Stutensee (DE)
(86) Internationale Anmeldenummer: PCT/EP2006/067080
(87) Internationale Veröffentlichungsnummer: WO 2007/042457

(56) Entgegenhaltungen:
- DE-A1- 10 211 275
- DE-A1-102004 032 129

## Beschreibung

Vorliegende Erfindung betrifft ein Verfahren zur Herstellung von Acrolein, oder Acrylsäure oder deren Gemisch aus Propan, bei dem man
A) dem Einlass in eine erste Reaktionszone A einen Reaktionsgasgemischeingangsstrom A zuführt, der durch Zusammenführung von wenigstens vier voneinander verschiedenen gasförmigen Ausgangsströmen 1, 2, 3 und 4 mit der Maßgabe erzeugt wurde, dass die drei gasförmigen Ausgangsströme 1, 2 und 3 Propan enthalten, der gasförmige Ausgangsstrom 4 molekularer Wasserstoff und der gasförmige Ausgangsstrom 3 Frischpropan ist,
   den Reaktionsgasgemischeingangsstrom A in der Reaktionszone A durch wenigstens ein Katalysatorbett führt, an welchem, gegebenenfalls unter Zufuhr weiterer Gasströme, durch partielle heterogen katalysierte Dehydrierung des Propan ein Propan und Propylen enthaltender Produktgasgemischstrom A gebildet wird,
   den Produktgasgemischstrom A durch den Auslass aus der ersten Reaktionszone A aus selbiger herausführt und dabei in zwei Produktgasgemisch A-Teilströme 1 und 2 mit identischer Zusammensetzung aufteilt und den Produktgasgemisch A-Teilstrom 1 in erster Kreisgasfahrweise als gasförmigen Ausgangsstrom 1 in die erste Reaktionszone A rückführt,
   den Produktgasgemisch A-Teilstrom 2 gegebenenfalls in eine erste Trennzone A führt, um in selbiger von in ihm enthaltenen, von Propan und Propylen verschiedenen Bestandteilen eine Teilmenge oder mehr abzutrennen, und dadurch einen verbleibenden, Propan und Propylen enthaltenden, Produktgasgemischstrom A' zu erzeugen,
B) den Produktgasgemischstrom A-Teilstrom 2 oder den Produktgasgemischstrom A' in einer zweiten Reaktionszone B zu Beschickung wenigstens eines Oxidationsreaktors verwendet und in dem wenigstens einen Oxidationsreaktor das im Produktgasgemisch A-Teilstrom 2 oder im Produktgasgemischstrom A' enthaltene Propylen einer selektiven heterogen katalysierten partiellen Gasphasenoxidation mit molekularem Sauerstoff zu einem Acrolein, oder Acrylsäure, oder deren Gemisch als Zielprodukt, nicht umgesetztes Propan und gegebenenfalls nicht umgesetztes Propylen sowie überschüssigen molekularen Sauerstoff enthaltenden Produktgasgemischstrom B unterwirft,
   den Produktgasgemischstrom B aus der Reaktionszone B herausführt und in einer zweiten Trennzone B im Produktgasgemischstrom B enthaltenes Zielprodukt abtrennt und von dem dabei verbleibenden, nicht umgesetztes Propan, molekularen Sauerstoff sowie gegebenenfalls nicht umgesetztes Propylen enthaltenden Restgas wenigstens eine nicht umgesetztes Propan, molekularen Sauerstoff und gegebenenfalls nicht umgesetztes Propylen enthaltende Teilmenge in zweiter Kreisgasfahrweise als gasförmigen Ausgangsstrom 2 mit der Maßgabe in die Reaktionszone A rückführt, dass man die gasförmigen Ausgangsströme 2, 3 und 4 sowie gegebenenfalls zusätzliche vom gasförmigen Ausgangsstrom 1 verschiedene gasförmige Ausgangsströme zu einem gasförmigen Treibstrahlgemischstrom vereinigt und anschließend mit dem gasförmigen Treibstrahlgemischstrom als Treibstrahl eine Strahlpumpe, die eine Treibdüse, eine Mischstrecke, einen Diffusor und einen Saugstutzen umfasst, betreibt, wobei die Förderrichtung des durch die Treibdüse über die Mischstrecke und den Diffusor entspannten Treibstrahls in den Einlass der ersten Reaktionszone A sowie die Saugwirkung des Saugstutzens in Richtung des den Produktgasgemischstrom A führenden Auslass der ersten Reaktionszone A weist und dabei durch den im Saugstutzen erzeugten Unterdruck unter Aufteilung des Produktgasgemischstroms A in die beiden Teilströme 1 und 2 den Produktgasgemisch A-Teilstrom 1 ansaugt, bei gleichzeitiger Vermischung mit dem Treibstrahl durch die Mischstrecke über den Diffusor transportiert und den dabei gebildeten Reaktionsgasgemischeingangsstrom A in den Einlass der ersten Reaktionszone A entlässt.

Acrylsäure ist als ein Partialoxidationsprodukt des Propylen ein bedeutendes Monomeres, das als solches oder in Form seiner Alkylester zur Erzeugung von z. B. als Klebstoffen geeigneten oder Wasser superabsorbierenden Polymerisaten Verwendung findet (vgl. z. B. WO 02/055469 und WO 03/078378). Acrolein ist ein bedeutendes Zwischenprodukt, beispielsweise für die Herstellung von Glutardialdehyd, Methionin, Folsäure und Acrylsäure.

Verfahren zur Herstellung von Acrolein und/oder Acrylsäure, bei denen man das Propylen durch partielle heterogen katalysierte Dehydrierung aus Propan erzeugt und im Beisein von nicht umgesetztem (inertem) Propan als Bestandteil eines Partialoxidationsgemischs einer heterogen katalysierten partiellen Gasphasenoxidation mit molekularem Sauerstoff zu Acrolein und/oder Acrylsäure enthaltenden Produktgasgemischen unterwirft, sind bekannt (vgl. z. B. DE-A 102 45 585 und den in dieser Schrift zitierten Stand der Technik).

Im Unterschied zur exotherm verlaufenden heterogen katalysierten Oxidehydrierung, die durch anwesenden Sauerstoff erzwungen und bei der intermediär kein freier Wasserstoff gebildet wird (der dem zu dehydrierenden Propan entrissene Wasserstoff wird unmittelbar als Wasser (H₂O) entrissen) bzw. nachweisbar ist, soll dabei in dieser Schrift unter einer heterogen katalysierten Dehydrierung eine ("konventionelle") Dehydrierung verstanden werden, deren Wärmetönung im Unterschied zur Oxidehydrierung endotherm ist (als Folgeschritt kann eine exotherme Wasserstoffverbrennung in die heterogen katalysierte Dehydrierung einbezogen sein) und bei der wenigstens intermediär freier molekularer Wasserstoff gebildet wird. Dazu bedarf es in der Regel anderer Reaktionsbedingungen und anderer Katalysatoren als zur Oxidehydrierung.

In entsprechender Weise wird in dieser Schrift unter Frischpropan Propan verstanden, das weder an einer Dehydrierung in der Reaktionszone A, noch an einer Partialoxidation von Propylen zu Acrolein und/oder Acrylsäure in der Reaktionszone B teilgenommen hat. Vorzugsweise hat es überhaupt noch an keiner chemischen Reaktion teilgenommen. In der Regel wird es in Form von Roh-Propan zugeführt, das vorzugsweise die Spezifikation gemäß DE-A 102 46 119 und DE-A 102 45 585 erfüllt, und das normalerweise in geringen Mengen auch von Propan verschiedene Komponenten enthält. Solches Roh-Propan ist beispielsweise nach in der DE-A 10 2005 022 798 beschriebenen Verfahren erhältlich. Normalerweise enthält Roh-Propan zu wenigstens ≥ 90 Gew.-% und vorzugsweise zu wenigstens ≥ 95 Gew.-% Propan.

Üblicherweise werden die vorstehend angesprochenen Verfahren des Standes der Technik so durchgeführt, dass man von dem nach der Zielproduktabtrennung aus dem Produktgasgemisch der Partialoxidation verbleibenden, nicht umgesetztes Propan, molekularen Sauerstoff sowie gegebenenfalls nicht umgesetztes Propylen enthaltenden Restgas, wenigstens eine nicht umgesetztes Propan, molekularen Sauerstoff und gegebenenfalls nicht umgesetztes Propylen enthaltende Teilmenge in Kreisgasfahrweise in die heterogen katalysierte Dehydrierung zurückführt.

Dabei wurde schon vorgeschlagen (z.B. DE-A 10 2004 032 129 und DE-A 10 2005 013 039), dass ein Gemisch aus Wasserdampf, Frischpropan und solchem Kreisgas als Reaktionsgasgemischeingangsgas der heterogen katalysierten Dehydrierung des Propan zum Propylen zugeführt wird. Dabei sollte die heterogen katalysierte Propandehydrierung zweckmäßig als Hordenreaktor verwirklicht sein, in welchem die Katalysatorbetten in günstiger Weise radial oder axial hintereinander angeordnet sind. Zweckmäßigerweise wird in einem solchen Hordenreaktor der Katalysatorfestbetttyp angewendet. In vorteilhafter Weise liegt die Anzahl der Katalysatorbetthorden in einem solchen Hordenreaktor bei drei. Dabei empfiehlt der Stand der Technik, die heterogen katalysierte partielle Propandehydrierung autotherm durchzuführen. Dazu wird dem Reaktionsgasgemisch hinter dem ersten durchlaufenen Katalysatorbett und zwischen den auf das in Strömungsrichtung erste Katalysator(fest)bett nachfolgenden Katalysator(fest)betten in begrenztem Umfang molekularer Sauerstoff (z. B. in Form von Luft) zugesetzt. So kann, in der Regel durch die Dehydrierkatalysatoren selbst katalysiert, eine begrenzte Verbrennung von im Verlauf der heterogen katalysierten Propandehydrierung gebildetem molekularem Wasserstoff (sowie gegebenenfalls in höchstens geringem Unfang von Propan) bewirkt werden, deren exotherme Wärmetönung die Dehydriertemperatur im wesentlich erhält (adiabate Reaktorgestaltung).

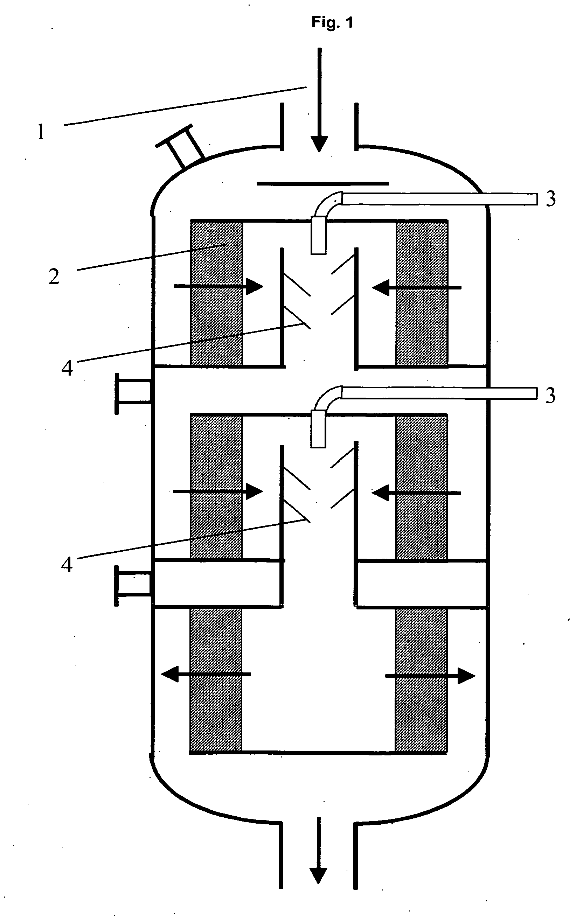

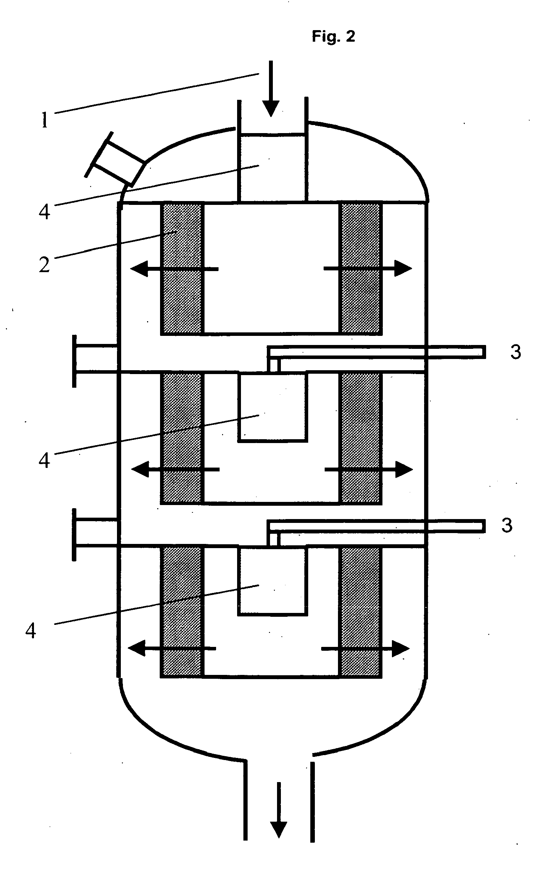

In den Vergleichsbeispielen 1, 3 und 4 der DE-A 10 2004 032 129 sowie im Ausführungsbeispiel der DE-A 10 2005 010 111 wird eine solche wie vorstehend beschriebene heterogen katalysierte partielle Propandehydrierung im Hordenreaktor (3 Katalysatorbetthorden) durch drei hintereinandergeschaltete Dehydrierrohrreaktoren simultiert. Anstelle einer solchen Hintereinanderschaltung von Rohrreaktoren kann in diesen Vergleichsbeispielen bzw. in diesem Ausführungsbeispiel aber auch ein (adiabater) Hordenreaktor gemäß den beiliegenden Figuren 1 und 2 verwendet werden (mit jeweils 3 Katalysatorfestbetthorden).

Im Hordenreaktortyp gemäß Figur 1 wird die Katalysatorschüttung (2) jeweils von außen nach innen durchströmt. Hingegen wird im Hordenreaktortyp gemäß Figur 2 die jeweilige Katalysatorschüttung (2) von innen nach außen durchströmt. Die Adressen (1) stehen jeweils für das Reaktionsgasgemischeingangsgas, (3) zeigt die Lufteinspeisung an und (4) sind Mischelemente.

Im übrigen sind folgende Reaktordaten, bezogen auf eine Propanströmung von 72280 kg/h im Reaktionsgasgemischeingangsgas, und eine Gesamtlufteinspeisung von 3496 kg/h zweckmäßig:

| | | |
|---|---|---|
| Reaktor Fig. 1: | Katalysatorgesamtmasse im Reaktor | = 30 t |
| | Katalysatormasse je Horde | = 10 t |
| | Höhe der Katalysatorschüttung je Horde | = 5,47 m |
| | Massendichte je Katalysatorschüttung | = 1200 kg/m³ |
| | Innenradius der Katalysatorschüttung | = 1,05 m |
| | Außenradius der Katalysatorschüttung | = 1,26 m |
| | Volumen der Katalysatorschüttung je Horde | = 8,33 m³ |
| | Durchmesser des Eintrittsrohres | = 1,4 m |
| | Reaktorinnendurchmesser | = 3,2 m |
| | Reaktorhöhe zylindrischer Schuss (ohne Hauben) | = 21 m |
| | Anzahl der Mischelemente | = 2 |
| | Eintrittsdruck des Reaktionsgemischeingangsgases | = 3,1 bar abs. |

Als Katalysator ist dabei jeweils der Katalysator des entsprechenden Vergleichsbeispiels bzw. des Ausführungsbeispiels zu verwenden. Das gleiche gilt für die Reaktionstemperaturen und die Zusammensetzung des Reaktionsgasgemischeingangsgases.

Bevorzugtes Reaktorkonstruktionsmaterial ist zweckmäßig für alle Reaktorteile Si-haltiger Edelstahl oder Stahl, z. B. solcher vom Typ 1.4841.

| | | |
|---|---|---|
| Reaktor Fig. 2: | Katalysatorgesamtmasse im Reaktor | = 30 t |
| | Katalysatormasse je Horde | = 10 t |
| | Höhe der Katalysatorschüttung je Horde | = 4,84 m |
| | Massendichte je Katalysatorschüttung | = 1200 kg/m³ |
| | Innenradius der Katalysatorschüttung | = 1,2 m |
| | Außenradius der Katalysatorschüttung | = 1,41 m |
| | Volumen der Katalysatorschüttung je Horde | = 8,33 m³ |
| | Durchmesser des Eintrittsrohres | = 1,4 m |
| | Reaktorinnendurchmesser | = 3,4 m |
| | Reaktorhöhe zylindrischer Schuss (ohne Hauben) | = 19 m |
| | Anzahl der Mischelemente | = 2 |
| | Eintrittsdruck des Reaktionsgemischeingangsgases | = 3,1 bar abs. |

Als Katalysator ist dabei jeweils der Katalysator des entsprechenden Vergleichsbeispiels bzw. des Ausführungsbeispiels zu verwenden. Das gleiche gilt für die Reaktionstemperaturen und die Zusammensetzung des Reaktionsgemischeingangsgases. Bevorzugtes Reaktorkonstruktionsmaterial ist zweckmäßig für alle Reaktorteile Si-haltiger Edelstahl oder Stahl, z. B. solcher vom Typ 1.4841.

Zweckmäßig wird die partielle heterogen katalysierte Dehydrierung des Propans dabei im wesentlichen auf die drei Katalysatorhorden verteilt so betrieben, dass der Umsatz des in den Reaktor geführten Propans, bezogen auf einmaligen Reaktordurchgang, ca. 20 mol.-% beträgt. Die erzielte Selektivität der Propylenbildung liegt dabei regelmäßig bei 90 mol.-%. Der maximale Umsatzbeitrag einer einzelnen Horde wandert mit zunehmender Betriebsdauer in Strömungsrichtung von vorne nach hinten. In der Regel wird die Katylsatorbeschickung regeneriert, bevor die in Strömungsrichtung dritte Horde den maximalen Umsatzbeitrag erbringt. Mit Vorteil erfolgt die Regenerierung dann, wenn die Verkokung aller Horden ein identisches Ausmaß erreicht hat.

Günstig ist es für die vorbeschriebene heterogen katalysierte partielle Dehydrierung des Propan ganz generell, wenn die Belastung der Katalysatorgesamtmenge (Summe über alle Betten) mit der Gesamtmenge aus Propan und Propylen ≥ 500 Nl/l·h und ≤ 20000 Nl/l·h beträgt (typische Werte sind 1500 Nl/l·h bis 2500 Nl/l·h). Die maximale Reaktionstemperatur innerhalb eines individuellen Katalysatorfestbetts wird dabei mit Vorteil bei 500°C bis 600°C gehalten. Mit besonderem Vorteil besteht das Reaktionsgasgemischeingangsgas bei vorbeschriebener heterogen katalysierter partieller Propandehydrierstufe im Hordenreaktor lediglich aus Frischpropan und dem aus der Partialoxidation in die Dehydrierung rückgeführten Kreisgas, das von der Partialoxidation herrührend eine ausreichende Menge an Wasserdampf enthält, um eine befriedigende Standzeit der Dehydrierkatalysatorbetten zu bedingen. D.h., die Vergleichsbeispiele und das Beispiel sind in den beschriebenen Hordenreaktoren auch dann so durchführbar, wenn auf den Zusatz des Extrawasserdampf in der Dehydrierung verzichtet wird.

Im übrigen gelten die in den Schriften DE-A 10 2005 009 885, DE-A 10 2005 010111, DE-A 10 2005 009 891, DE-A 10 2005 013 039 und DE-A 10 2004 032 129 zu solchen Verfahrensweisen gemachten Aussagen. Unter der Belastung eines einen Reaktionsschritt katalysierenden Katalysatorbetts mit Reaktionsgasgemisch wird auch in dieser Schrift die Menge an Reaktionsgasgemisch in Normlitern (= Nl; das Volumen in Litern, das die entsprechende Reaktionsgasgemischmenge bei Normalbedingungen (0°C, 1 bar) einnehmen würde) verstanden, die pro Stunde durch einen Liter Katalysatorbett (z. B. Katalysatorfestbett) geführt wird. Die Belastung kann aber auch nur auf einen Bestandteil des Reaktionsgasgemischs bezogen sein. Dann ist es die Menge dieses Bestandteils in Nl/l·h, die pro Stunde durch einen Liter des Katalysatorbetts geführt wird (reine Inertmaterialschüttungen werden nicht zum Katalysatorfestbett gerechnet).

Die Belastung kann auch nur auf die in einem Katalysatorbett, das den eigentlichen Katalysator mit Inertmaterial verdünnt enthalten kann, enthaltene Menge an Katalysator bezogen sein (dies wird dann explizit vermerkt).

Nachteilig am beschriebenen Verfahren des Standes der Technik ist, dass nahezu alle Katalysatoren, die die Dehydrierung des Propan katalysieren, auch die Verbrennung (vollständige Oxidation von Propan und Propylen zu Kohlenoxiden und Wasserdampf) von Propan und Propylen mit molekularem Sauerstoff katalysieren und molekularer Sauerstoff normalerweise aber bereits im in die heterogen katalysierte partielle Dehydrierung des Propan rückgeführten Kreisgas aus der Partialoxidation enthalten ist. Dies rührt daher, dass aus Gründen einer erhöhten Katalysatorstandzeit bei der heterogen katalysierten Partialoxidation der molekulare Sauerstoff, gemessen an der Stöchiometrie der Partialoxidation, normalerweise im Überschuss verwendet wird. Die katalysierte Verbrennungsreaktion dieses molekularen Sauerstoff mit im Reaktionsgemischeingangsgas enthaltenem Propan und/oder Propylen mindert die Selektivität der Propenbildung im Rahmen der heterogen katalysierten partiellen Propandehydrierung.

Es wurde deshalb auch schon vorgeschlagen (siehe DE-A 102 11 275), im Rahmen einer mehrstufigen Herstellung von Acrolein und/oder Acrylsäure aus Propan die heterogen katalysierte partielle Propandehydrierung so auszuführen, dass man das der Dehydrierzone entnommene Produktgas in zwei Teilmengen identischer Zusammensetzung aufteilt, um nur eine der beiden Teilmengen der Partialoxidation zuzuführen, während die andere Teilmenge als Bestandteil des Reaktionsgasgemischeingangsgases in die Dehydrierung rückgeführt wird. Der in diesem aus der Dehydrierung selbst kommenden Kreisgas enthaltene molekulare Wasserstoff, soll dann das im Reaktionsgasgemischeingangsgas enthaltene Propan und gegebenenfalls Propylen vor dem in diesem Eingangsgas gleichfalls enthaltenen molekularen Sauerstoff schützen. Dieser Schutz fußt darauf, dass die normalerweise durch die selben Katalysatoren heterogen katalysierte Verbrennung von molekularem Wasserstoff zu Wasser gegenüber der Vollbrennung von Propan und/oder Propylen kinetisch bevorzugt ist.

In der DE-A 102 11 275 wird auch bereits die Dehydrierkreisgasführung mittels des Strahlpumpenprinzips offenbart (sie wird auch als Schlaufenfahrweise bezeichnet). Ferner wird in dieser Schrift die Möglichkeit angesprochen, dem Reaktionsgasgemisch in der Propandehydrierung zusätzlich molekularen Wasserstoff zuzusetzen. Über das Erfordernis, den molekularen Wasserstoff in den Treibstrahl für die Strahlpumpe in bestimmter Zufuhrhierarchie zuzudosieren, schweigt sich die DE-A 102 11 275 aus.

Die DE-A 10 2004 032 129 und DE-A 10 2005 013 039 schlägt vor, die Rückführung des aus der heterogen katalysierten Partialoxidation herrührenden, molekularen Sauerstoff enthaltenden, Kreisgases nicht in das Reaktionsgasgemischeingangsgas für die heterogen katalysierte partielle Propandehydrierung hinein vorzunehmen. Vielmehr soll die Rückführung erst noch einem bestimmten Dehydrierumsatz in das Reaktionsgasgemisch der Dehydrierung erfolgen. Dabei wird auch in der DE-A 10 2004 032 129 auch vorgeschlagen, vorab dieser Rückführung dem Reaktionsgasgemisch der Dehydrierung zusätzlich externen molekularen Wasserstoff zuzusetzen. Des weiteren wird auch in der DE-A 10 2004 032 129 die Schlaufenfahrweise für die Dehydrierung propagiert. Treibstrahl ist dabei ausschließlich das aus der Partialoxidation in die Dehydrierung rückgeführte Kreisgas.

Diese Betriebsweise ist jedoch insofern nachteilig, als bei ihr der extern zudosierte molekulare Wasserstoff noch in signifikanter Teilmenge als Bestandteil des Produktgasgemischs aus der Dehydrierung in die Partialoxidation geführt wird, ohne dass sein Schutzpotential zuvor umfassend genutzt worden wäre. Des weiteren bringt die Strahlpumpe auch die Teilmenge des Produktgasgemischs aus der partiellen Dehydrierung auf einen erhöhten Druck, die in die Partialoxidation entlassen wird. Vorab der Partialoxidation ist jedoch normalerweise in jedem Fall eine zusätzliche Kompression des entsprechenden Reaktionsgasgemischausgangsgases mittels eines separaten Kompressor erforderlich, um den mit der Partialoxidation einhergehenden Druckverlust auszugleichen. Unter Druck wird in der Regel auch die Wandlung des Produktgasgemischstroms A-Teilstrom 2 in den Produktgasgemischstrom A' durchgeführt. Vor diesem Hintergrund ist eine wie beschrieben einhergehenden Druckbelastung der Strahlpumpe wenig zweckmäßig.

Die DE-A 10 2005 009 885 empfiehlt vor diesem Hintergrund im Ausführungsbeispiel II eine Schlaufenfahrweise, bei dem das Reaktionsgasgemischeingangsgas für die heterogen katalysierte partielle Dehydrierung des Propans zusammengesetzt ist aus Kreisgas, das aus der Partialoxidation rückgeführt wird, aus Frischpropan, aus externem molekularem Wasserstoff, aus einer minimalen Menge an externem Wasserdampf und aus der Dehydrierung selbst rückgeführtem Kreisgas (auf den externen Wasserdampf könnte auch verzichtet werden). Als Treibstrahl wird ein Gemisch aus Frischpropan, externem molekularem Wasserstoff, Kreisgas aus der Partialoxidation und externem Wasserdampf verwendet. Hinsichtlich einer einzuhaltenden Zudosierungsabfolge bei der Treibstrahlerzeugung schweigt sich die DE-A 10 2005 009 885 aus. Dies rührt daher, dass die Anmelderin zum damaligen Zeitpunkt, zu dem die betriebene Versuchsanlage aus Edelstahl neu war, keine Hinweise auf das Erfordernis einer speziellen Zudosierungsabfolge hatte. Inzwischen hat sich jedoch gezeigt, dass im Verlauf längerer Betriebszeit sich in Anlagen aus Edelstahl oder aus konventionellem Stahl (d. h., allgemein aus Stahl) in geringsten Mengen ansetzender Flugrost die Verbrennung von molekularem Wasserstoff mit molekularem Sauerstoff katalysiert. Dies ist insofern von Nachteil, als die dabei gebildete Verbrennungswärme zumindest teilweise nicht dort zur Geltung kommt, wo sie benötigt wird, nämlich bei der endothermen Dehydrierung. Vielmehr verpufft sie selbst bei angestrebter adiabater Dehydrierung wenigstens teilweise, da eine ideale adiabate Reaktionsvorrichtung nicht realisierbar ist. Dies bedingt entweder die Notwendigkeit einer Zufuhr von externer Wärme, mit der in der Regel an den Wärmeübertragungsflächen unerwünschte Crackprozesse der involvierten Kohlenwasserstoffe einhergehen, oder es resultieren Dehydrierumsätze die signifikant vermindert sind. Beides ist von Nachteil.

Die Aufgabe der vorliegenden Erfindung bestand daher darin, ein verbessertes Verfahren zur Herstellung von Acrolein, oder Acrylsäure oder deren Gemisch aus Propan zur Verfügung zu stellen, das die beschriebenen Nachteile entweder nicht mehr, oder allenfalls nur noch in verminderter Form aufweist.

Demgemäß wurde ein Verfahren zur Herstellung von Acrolein, oder Acrylsäure oder deren Gemisch aus Propan, bei dem man
A) dem Einlass in eine erste Reaktionszone A einen Reaktionsgasgemischeingangsstrom A zuführt, der durch Zusammenführung von wenigstens vier voneinander verschiedenen gasförmigen Ausgangsströmen 1, 2, 3 und 4 mit der Maßgabe erzeugt wurde, dass die drei gasförmigen Ausgangsströme 1, 2 und 3 Propan enthalten, der gasförmige Ausgangsstrom 4 molekularer Wasserstoff und der gasförmige Ausgangsstrom 3 Frischpropan ist,
   den Reaktionsgasgemischeingangsstrom A in der Reaktionszone A durch wenigstens ein Katalysatorbett führt, an welchem, gegebenenfalls unter Zufuhr weiterer Gasströme, durch partielle heterogen katalysierte Dehydrierung des Propan ein Propan und Propylen enthaltender Produktgasgemischstrom A gebildet wird,
   den Produktgasgemischstrom A durch den Auslass aus der ersten Reaktionszone A aus selbiger herausführt und dabei in zwei Produktgasgemisch A-Teilströme 1 und 2 mit identischer Zusammensetzung aufteilt und den Produktgasgemisch
   A-Teilstrom 1 in erster Kreisgasfahrweise als gasförmigen Ausgangsstrom 1 in die erste Reaktionszone A rückführt,
   den Produktgasgemisch A-Teilstrom 2 gegebenenfalls in eine erste Trennzone A führt, um in selbiger von in ihm enthaltenen, von Propan und Propylen verschiedenen Bestandteilen eine Teilmenge oder mehr abzutrennen, und dadurch einen verbleibenden, Propan und Propylen enthaltenden, Produktgasgemischstrom A' zu erzeugen,
B) den Produktgasgemischstrom A-Teilstrom 2 oder den Produktgasgemischstrom A' in einer zweiten Reaktionszone B zur Beschickung wenigstens eines Oxidationsreaktors verwendet und in dem wenigstens einen Oxidationsreaktor das im Produktgasgemisch A-Teilstrom 2 oder im Produktgasgemischstrom A' enthaltene Propylen einer selektiven heterogen katalysierten partiellen Gasphasenoxidation mit molekularem Sauerstoff zu einem Acrolein, oder Acrylsäure, oder deren Gemisch als Zielprodukt, nicht umgesetztes Propan und gegebenenfalls nicht umgesetztes Propylen sowie überschüssigen molekularen Sauerstoff enthaltenden Produktgasgemischstrom B unterwirft,
   den Produktgasgemischstrom B aus der Reaktionszone B herausführt und in einer zweiten Trennzone B im Produktgasgemischstrom B enthaltenes Zielprodukt abtrennt und von dem dabei verbleibenden, nicht umgesetztes Propan, molekularen Sauerstoff sowie gegebenenfalls nicht umgesetztes Propylen enthaltenden Restgas wenigstens eine nicht umgesetztes Propan, molekularen Sauerstoff und gegebenenfalls nicht umgesetztes Propylen enthaltende Teilmenge in zweiter Kreisgasfahrweise als gasförmigen Ausgangsstrom 2 mit der Maßgabe in die Reaktionszone A rückführt, dass man die gasförmigen Ausgangströme 2, 3 und 4 sowie gegebenenfalls zusätzliche vom gasförmigen Ausgangsstrom 1 verschiedene gasförmige Ausgangsströme zu einem gasförmigen Treibstrahlgemischstrom vereinigt und anschließend mit dem gasförmigen Treibstahlgemischstrom als Treibstrahl eine Strahlpumpe, die eine Treibdüse, eine Mischstrecke, einen Diffusor und einen Saugstutzen umfasst, betreibt, wobei die Förderrichtung des durch die Treibdüse über die Mischstrecke und den Diffuser entspannten Treibstrahls in den Einlass der ersten Reaktionszone A sowie die Saugwirkung des Saugstutzens in Richtung des den Produktgasgemischstrom A führenden Auslass der ersten Reaktionszone A weist und dabei durch den im Saugstutzen erzeugten Unterdruck unter Aufteilung des Produktgasgemischstroms A in die beiden Teilströme 1 und 2 den Produktgasgemisch A-Teilstrom 1 ansaugt, bei gleichzeitiger Vermischung mit dem Treibstrahl durch die Mischstrecke über den Diffusor transportiert und den dabei gebildeten Reaktionsgasgemischeingangsstrom A in den Einlass der ersten Reaktionszone A entlässt, gefunden,
   das dadurch gekennzeichnet ist, dass man zunächst die gasförmigen Ausgangsströme 2 und 3 sowie gegebenenfalls zusätzliche, von den gasförmigen Ausgangsströmen 1 und 4 verschiedene gasförmige Ausgangsströme in beliebiger Abfolge zu einem gasförmigen Ausgangsgemischstrom vereinigt und erst dem gasförmigen Ausgangsgemischstrom unter Ausbildung des gasförmigen Treibstrahlgemischstroms den gasförmigen Ausgangsstrom 4 beifügt.

Abgesehen vom kennzeichnenden Teil kann das erfindungsgemäße Verfahren wie die in den Schriften EP-A 117 146, US-A 3,161,670, DE-A 33 13 573, WO 01/96270, DE-A 103 160 39, DE-A 10 2005 013 039, DE-A 10 2004 032 129, DE-A 102 11 275, DE-A 102 45 585, DE-A 10 2005 009 891, DE-A 10 2005 010 111, DE-A 10 2005 022 798 und DE-A 10 2005 009 885 beschriebenen entsprechenden Verfahren durchgeführt werden.

Erfindungsgemäß bevorzugt erfolgt die Hinzufügung des gasförmigen Ausgangsstroms 4 zum gasförmigen Ausgangsgemischstrom (unter Ausbildung des Treibstrahlgemischstroms) innerhalb möglichst kurzer Zeit. Ferner erfolgt die Hinzufügung des gasförmigen Ausgangsstroms 4 zum gasförmigen Ausgangsgemischstrom (unter Ausbildung des Treibstrahlgemischstroms) so, dass vom Zeitpunkt der Ausbildung des Treibstrahlgemischstroms bis zu dem Zeitpunkt, an dem der Reaktionsgasgemischeingangsstrom A das (in Strömungsrichtung) erste Katalysatorbett der Reaktionszone A mit Dehydrierkatalysator erreicht, nicht mehr als 30 Sekunden, vorzugsweise nicht mehr als 20 oder als 10 Sekunden, mit Vorteil nicht mehr als 7 Sekunden, besonders bevorzugt nicht mehr als 5 Sekunden, ganz besonders bevorzugt nicht mehr als 3 Sekunden und am Besten nicht mehr als 1 bzw. 0,5 oder 0,1 Sekunden vergeht.

Als Verfahren zur Abtrennung von im Produktgasgemischstrom B enthaltenem Zielprodukt kommen für das erfindungsgemäße Verfahren prinzipiell alle im Stand der Technik diesbezüglich bekannten Verfahren in Betracht. Sie sind im wesentlichen dadurch gekennzeichnet, dass man das Zielprodukt z.B. durch absorptive und/oder kondensative Maßnahmen aus der gasförmigen in die kondensierte Phase überführt. Als Absorptionsmittel kommen dabei z. B. Wasser, wässrige Lösung und/oder organische Lösungsmittel in Betracht. Im Rahmen dieser "Kondensation" des Zielproduktes verbleibt normalerweise ein nicht in die kondensierte Phase übergehendes Restgas, das die vergleichsweise schwierig zu kondensierenden Bestandteile des Produktgasgemischstroms B umfasst. Dies sind üblicherweise vor allem diejenigen Komponenten, deren Siedepunkt bei Normaldruck (1 bar) ≤ -30°C beträgt (ihr Gesamtanteil am Restgas beträgt in der Regel ≥ 70 Vol.-%, häufig ≥ 80 Vol.-% und vielfach ≥ 90 Vol.-%). Dazu gehören in erster Linie nicht umgesetztes Propan, im Produktgasgemischstrom B verbliebener überschüssiger molekularer Sauerstoff sowie gegebenenfalls nicht umgesetztes Propylen. Zusätzlich wird das Restgas in der Regel inerte Verdünnungsgase wie z.B. N₂, CO₂, Edelgase (He, Ne, Ar etc.), CO sowie in geringem Umfang auch Acrylsäure, Acrolein und/oder H₂O (der Wasserdampfanteil am Restgas kann bis zu 25 Vol.-%, häufig bis zu 20 Vol.-%, oder bis zu 10 Vol.-%, vielfach aber auch unter 10 Vol.-% oder unter 5 Vol.-% betragen) enthalten. Dieses vorgenannte Restgas bildet (bezogen auf die darin enthaltene Menge an Propan) normalerweise die Hauptmenge (üblicherweise wenigstens 80%, bzw. wenigstens 90%, oder wenigstens 95% oder mehr) des in der Trennzone B gebildeten Restgases und wird daher in dieser Schrift u.a. auch als Hauptrestgas bezeichnet.

Erfindungsgemäß wird normalerweise wenigstens eine nicht umgesetztes Propan, molekularen Sauerstoff, sowie gegebenenfalls nicht umgesetztes Propylen enthaltende Teilmenge dieses Restgases (Hauptrestgases) in Kreisgasfahrweise als gasförmiger Ausgangsstrom 2 in die Reaktionszone A rückgeführt werden. Erfindungsgemäß zweckmäßig wird vielfach die Gesamtmenge dieses Restgases in die Reaktionszone A als gasförmiger Ausgangsstrom 2 rückgeführt.

Insbesondere dann, wenn die Kondensation des Zielproduktes durch Absorption mittels eines organischen Lösungsmittels erfolgt, fällt in der Trennzone B in der Regel wenigstens ein zweites nicht umgesetztes Propan sowie gegebenenfalls nicht umgesetztes Propylen enthaltendes restliches Gas an (bezogen auf darin enthaltendes Propan ist seine Menge im Vergleich zur Hauptrestgasmenge normalerweise wesentlich geringer). Dies ist darauf zurückzuführen, dass die sich bildende kondensierte Phase in gewissem Umfang auch nicht umgesetztes Propan sowie gegebenenfalls nicht umgesetztes Propylen aufnimmt.

Im weiteren Verlauf der extraktiven, destillativen, kristallisativen und/oder desorptiven Abtrennung des Zielproduktes aus der kondensierten Phase, wird dieses nicht umgesetzte Propan sowie gegebenenfalls Propylen normalerweise als Bestandteil wenigstens einer weiteren Gasphase rückgewonnen und beim erfindungsgemäßen Verfahren vorzugsweise ebenfalls in die Reaktionszone A rückgeführt.

Dies kann z. B. im Gemisch mit dem Hauptrestgas erfolgen (in dieser Schrift dann Gesamtrestgas genannt). Es kann aber auch in Form eigenständiger in die Reaktionszone A rückzuführender Gasströme erfolgen. Selbstverständlich kann diese Rückfuhr in die Reaktionszone A auch als ein weiterer gasförmiger Ausgangsstrom erfolgen. Diese eigenständigen rückzuführenden Gasströme können an Sauerstoff frei, oder auch Sauerstoff enthaltend (Nebenrestgas) sein (z. B. wenn er durch Strippen mittels Luft oder am Kopf einer mittels Luft als Polymerisationsinhibitor gespülten Rektifikationskolonne anfällt).

Sowohl Hauptrestgas, Gesamtrestgas als auch Nebenrestgas bilden im Sinne dieser Erfindung als gasförmigen Ausgangsstrom 2 in die Reaktionszone A rückführbares nicht umgesetztes Propan, molekularen Sauerstoff und gegebenenfalls nicht umgesetztes Propylen enthaltendes Restgas. An molekularem Sauerstoff freies in der Trennzone B anfallendes nicht umgesetztes Propan sowie gegebenenfalls nicht umgesetztes Propylen enthaltendes restliches Gas kann erfindungsgemäß im Gemisch mit Hauptrestgas und/oder Nebenrestgas (d. h., z. B. als Bestandteil von Gesamtrestgas) z. B. als Bestandteil des gasförmigen Ausgangsstroms 2 und/oder auch eigenständig (in diesem Fall handelt es sich nicht um in die Reaktionszone A im Sinne der Erfindung rückgeführtes Restgas) in die Reaktionszone A rückgeführt werden. Im letzteren Fall kann diese Rückführung ohne jedwede Beschränkung, d. h., z. B. auch als ein weiterer gasförmiger Ausgangsstrom erfolgen. Insbesondere dann, wenn man beim erfindungsgemäßen Verfahren in einer ersten Trennzone A vom Produktgasgemisch A-Teilstrom 2 im wesentlichen alle in ihm enthaltenen, von Propan und Propylen verschiedenen Bestandteile abtrennt und anschließend den dabei resultierenden Produktgasgemischstrom A' zur Beschickung des wenigstens einen Oxidationsreaktors verwendet, wird man beim erfindungsgemäßen Verfahren im wesentlichen die Gesamtmenge der in der Trennzone B anfallenden, nicht umgesetztes Propan sowie gegebenenfalls nicht umgesetztes Propylen enthaltenden Gasströme in die Reaktionszone A rückführen, und dies vorzugsweise als Bestandteil von Gesamtrestgas als gasförmigem Ausgangsstrom 2. Teilmengen könnten (wie z. B. in der DE-A 10 2004 032 129 beschrieben) gegebenenfalls aber auch für andere Zwecke, wie z. B. zur Energieerzeugung und/oder Synthesegasherstellung und/oder als Verdünnungsgas in der Reaktionszone B weiterverwendet werden. In der Regel wird man im vorbeschriebenen Fall jedoch wenigstens die Hälfte oder zwei Drittel (d. h., 50 Vol.-% oder 66,6 Vol.-%), bevorzugt wenigstens drei Viertel und ganz besonders bevorzugt die Gesamtmenge des vorgenannten in der Trennzone B anfallenden (jeweils individuell bezüglich des Haupt- und/oder Neben- bzw. Gesamt-) Restgases in die Reaktionszone A, erfindungsgemäß bevorzugt als Bestandteil des gasförmigen Ausgangsstroms 2, rückführen. Fällt in der Trennzone B nur ein nicht umgesetztes Propan, molekularen Sauerstoff und nicht umgesetztes Propylen enthaltender Restgasstrom an (dies ist häufig der Regelfall), wird dieser, insbesondere dann, wenn man beim erfindungsgemäßen Verfahren in einer ersten Trennzone A vom Produktgasgemisch A-Teilstrom 2 im wesentlichen alle in ihm enthaltenen, von Propan und Propylen verschiedenen Bestandteile abtrennt und anschließend den dabei resultierenden Produktgasgemischstrom A' zur Beschickung wenigstens eines Oxidationsreaktors verwendet, erfindungsgemäß bevorzugt vollständig (gegebenenfalls abzüglich einer in die Reaktionszone B als Verdünnungsgas geführten Teilmenge von identischer Zusammensetzung) als gasförmiger Ausgangsstrom 2 in die Reaktionszone A rückgeführt. Er kann dann aber auch in zwei Teilmengen identischer Zusammensetzung aufgeteilt und, wie vorstehend beschrieben, nur eine Teilmenge als gasförmiger Ausgangsstrom 2 in die Reaktionszone A rückgeführt und die andere Teilmenge anderweitig weiterverwendet werden. Fällt in der Trennzone B mehr als ein solcher Restgasstrom an, so können diese Restgasströme (wie bereits erwähnt) erfindungsgemäß gemeinsam (z. B. zusammengeführt) als gasförmiger Ausgangsstrom 2 in die Reaktionszone A rückgeführt werden. Selbstverständlich kann die Rückführung dieser Restgasströme in die Reaktionszone A aber auch einzeln erfolgen.

Auch kann eine Teilmenge des Restgases nicht als gasförmiger Ausgangsstrom 2 sondern erst längs des Reaktionspfads der heterogen katalysierten Dehydrierung des Propans in der Reaktionszone A in die Reaktionszone A rückgeführt werden. Unter dem Reaktionspfad der heterogen katalysierten Dehydrierung des Propans in der ersten Reaktionszone A soll dabei der Strömungsweg des im Reaktionsgasgemischeingangsstrom A enthaltenen Propan durch die Reaktionszone A in Abhängigkeit vom dehydrierenden Umsatz (der Umsatz in der heterogen katalysierten Dehydrierung) dieses Propans verstanden.

Normalerweise besteht als gasförmiger Ausgangsstrom 2 in die Reaktionszone A rückgeführtes Restgas beim erfindungsgemäßen Verfahren zu ≥ 70 Vol.-%, häufig zu ≥ 80 Vol.-% und vielfach zu ≥ 90 Vol.-%, meist zu ≥ 95 Vol.-%, oder zu ≥ 98 Vol.-% aus Bestandteilen, deren Siedepunkt bei Normaldruck (1 bar) ≤ -30°C beträgt.

Insbesondere dann, wenn man beim erfindungsgemäßen Verfahren in einer ersten Trennzone A vom Produktgasgemisch A-Teilstrom 2 im wesentlichen alle in ihm enthaltenen, von Propan und Propylen verschiedenen Bestandteile abtrennt und anschließend den dabei resultierenden Produktgasgemischstrom A' zur Beschickung wenigstens eines Oxidationsreaktors verwendet, umfasst die Zusammensetzung des gasförmigen Ausgangsstromes 2 in typischer Weise:
0 bis 2 Vol.-%, vielfach 0 bis 1 Vol.-%, häufig 0 bis 0,5 Vol.-% Propen;
0 bis 2 Vol.-%, vielfach 0 bis 1 Vol.-%, häufig 0 bis 0,5 Vol.-% Acrolein;
0 bis 0,5 Vol.-%, vielfach 0 bis 0,1 Vol.-%, häufig 0 bis 0,05 Vol.-% Acrylsäure;
0 bis 4 Vol.-%, vielfach 0 bis 2 Vol.-%, häufig 0 bis 1,5 Vol.-% COₓ;
10 bis 50 Vol.-%, vielfach 20 bis 30 Vol.-% Propan;
0 bis 70 Vol.-%, vielfach 40 bis 70 Vol.-% N₂;
1 bis 10 Vol.-%, vielfach 2 bis 5 Vol.-%, häufig 2,5 bis 3,5 Vol.-% O₂ und
> 0 bis 15 Vol.-% H₂O.

Häufig weist der gasförmige Ausgangsstrom 2 beim erfindungsgemäßen Verfahren eine Temperatur von 50 bis 200°C, bzw. von 70 bis 130°C sowie einen Druck von 1,5 bis 5 bar, vorzugsweise von 3 bis 4 bar auf.

Typische Temperaturen für den gasförmigen Ausgangsstrom 3 liegen bei 0 bis 50°C, häufig bei 5 bis 20°C, bei Drücken von 3 bis 6 bar bzw. 4 bis 5 bar.

Gemäß der erfindungsgemäßen Verfahrensweise muss der gasförmige Ausgangsstrom 4 "molekularer Wasserstoff" sein. Darunter sollen in dieser Schrift Gasströme verstanden werden, die entweder nur aus molekularem Wasserstoff oder die zu wenigstens 50 Vol,-%, vorzugsweise zu wenigstens 60 Vol.-%, oder zu wenigstens 70 Vol.-%, oder zu wenigstens 80 Vol.-%, oder zu wenigstens 90 Vol.-%, oder zu wenigstens 95 Vol.-% bzw. zu wenigstens 98 Vol.-% bzw. zu wenigstens 99 Vol.-% aus molekularem Wasserstoff und in der jeweiligen Restmenge aus einem Inertgas bestehen.
Als Inertgas soll in dieser Schrift ganz generell ein Reaktionsgasbestandteil verstanden werden, der sich unter den Bedingungen der entsprechenden Reaktion (im vorgenannten Fall der heterogen katalysierten Dehydrierung) im wesentlich als inert verhält und - jeder inerte Reaktionsgasbestandteil für sich betrachtet - zu mehr als 95 mol.-%, vorzugsweise zu mehr als 99 mol.-% chemisch unverändert erhalten bleibt. Beispiele für solche Inertgase sind N₂, Edelgase, CO₂ oder auch Wasserdampf.

Erfindungsgemäß zweckmäßig weist der gasförmige Ausgangsstrom 4 beim erfindungsgemäßen Verfahren eine Temperatur von 20 bis 100°C, häufig 40 bis 60°C und einen Druck im Bereich von 1 bis 5 bar auf.

Vorteilhaft ist ein erfindungsgemäßes Verfahren, bei dem 60 bis 90 mol.-%, vorzugsweise 75 bis 85 mol.-% des im Reaktionsgasgemischeingangstrom A enthaltenen molekularen Wasserstoff dem Produktgasgemisch A-Teilstrom 1 (gasförmiger Ausgangsstrom 1) entstammt und die verbleibenden 10 bis 40 mol-%, bzw. 15 bis 25 mol.-% dem gasförmigen Ausgangsstrom 4.

Als über die gasförmigen Ausgangsströme 1 bis 4 hinausgehende gasförmige Ausgangsströme kommt insbesondere Wasserdampf bzw. Sprühnebel aus feinteiligen Wassertröpfchen in Betracht. In zweckmäßiger Weise weist ein solcher, z.B. gasförmiger Ausgangsstrom 5 aus Wasserdampf eine Temperatur von 100 bis 200°C, häufig 120 bis 160°C, und einen Druck von 1 bar bis 4 bar auf.

Erfindungsgemäß wird man einen solchen aus Wasserdampf bestehenden gasförmigen Ausgangsstrom 5 beim erfindungsgemäßen Verfahren im gasförmigen Ausgangsgemischstrom integrieren, bevor man zu letzterem den gasförmigen Ausgangsstrom 4 hinzufügt.

Die vorgenannte Integration erfolgt dabei zweckmäßig so, dass man zum gasförmigen Ausgangsstrom 2 zunächst den gasförmigen Ausgangsstrom 5 und zu dem dabei resultierenden gasförmigen Gemisch den gasförmigen Ausgangsstrom 3 hinzufügt.

Der dabei erhaltene Ausgangsgemischstrom wird mit Vorteil erst dann durch einen indirekten Wärmeaustauscher geführt, um in selbigem den Produktgasgemisch A-Teilstrom 2 abzukühlen (z.B. von 500 bis 600°C auf 150 bis 350°C) und gleichzeitig den Ausgangsstrom zu erwärmen (z. B. von 20 bis 200°C, auf 350 bis 530°C). Das Beisein von Wasserdampf mindert das Risiko einer mit der Erwärmung einhergehenden Verkokung. Danach erfolgt dann die erfindungsgemäß erforderlich Vereinigung des gasförmigen Ausgangsstroms 4 mit dem wie beschrieben auf Temperatur gebrachten Ausgangsgemischstrom unter Ausbildung des Treibstrahlgemischstroms. Letzterer weist erfindungsgemäß vorteilhaft eine Temperatur von 350 bis 550°C und einen Druck von 2 bis 5 bar auf.

Die Mitverwendung von Wasserdampf als gasförmigem Ausgangsstrom 5 ist insbesondere für die Katalysatorstandzeit in der heterogen katalysierten partiellen Dehydrierung von Vorteil, wie im folgenden noch beschrieben werden wird. Erfindungsgemäß vorteilhaft wird man jedoch auf die Mitverwendung eines solchen gasförmigen Ausgangsstroms 5 möglichst verzichten und es bei dem im gasförmigen Ausgangsstrom 2 mit Vorteil enthaltenen, von der im Rahmen der Partialoxidation erfolgenden Reaktionswasserbildung herrührenden Wasserdampf belassen. Wasserdampfgehalte im Reaktionsgasgemischeingangsstrom A von 1 bis 20 oder bis 15 bzw. bis 10 Vol.-%, häufig 4 bis 6 Vol.-%, haben sich erfindungsgemäß als zweckmäßig erwiesen.

Insbesondere dann, wenn man beim erfindungsgemäßen Verfahren den Produktgasgemisch A-Teilstrom 2 in einer erfindungsgemäß weniger bevorzugten Variante als solchen zur Beschickung des wenigstens einen Oxidationsreaktor verwendet, ist es erfindungsgemäß zweckmäßig, aus dem in der Trennzone B anfallenden Restgas wenigstens eine Teilmenge der in ihm enthaltenen von Propan, molekularem Sauerstoff und gegebenenfalls Propylen verschiedenen Bestandteile abzutrennen, bevor selbiges zur Bildung des gasförmigen Ausgangsstroms 2 herangezogen wird.

Entsprechende Abtrennungen sind z. B. in der EP-A 117 146, der US-A 3,161,670, der DE-A 33 13 573, der DE-A 103 16 039 und in der DE-A 102 45 585 beschrieben.

Für das erfindungsgemäße Verfahren ist es im Rahmen der in der Reaktionszone A anzuwendenden Schlaufenfahrweise günstig, wenn die Menge des Produktgasgemisch A-Teilstroms 1, bezogen auf die Gesamtmenge das Produktgasgemischstroms A, 25 bis 75 Vol.-% bzw. 30 bis 70 Vol.-%, vorteilhaft 40 bis 60 Vol.-% und besonders bevorzugt 50 Vol.-% beträgt.

Der Propanumsatz beim Durchgang des Reaktionsgasgemischeingangsstroms A, der sich erfindungsgemäß bevorzugt lediglich aus den gasförmigen Ausgangsströmen 1, 2, 3 und 4 sowie gegebenenfalls einem gasförmigen Ausgangsstrom 5 aus Wasserdampf konstituiert, kann beim erfindungsgemäßen Verfahren (bezogen auf den einmaligen Durchgang des Reaktionsgasgemischeingangsstroms A durch die Reaktionszone A sowie bezogen auf die Gesamtmenge aus im Reaktionsgasgemischeingangsstrom A enthaltenem Frischpropan und aus dem gasförmigen Ausgangsstrom 2 herrührendem Propan) 20 bis 30 mol-% betragen. Besonders günstig ist das erfindungsgemäße Verfahren jedoch dann, wenn der vorgenannte Propanumsatz 30 bis 60 mol-%, vorzugsweise 35 bis 55 mol-% und besonders bevorzugt 35 bis 45 mol-% beträgt.

Für die Realisierung der vorgenannten Propanumsätze ist es günstig, die partielle heterogen katalysierte Propandehydrierung in der Reaktionszone A bei einem Arbeitsdruck von 0,3 bis 10 bar, bzw. vorteilhaft bis 3 bar durchzuführen. Ein Beisein von Wasserdampf ermöglicht über die Wärmekapazität des Wassers einen Teil der Auswirkung der Endothermie der Dehydrierung auszugleichen und andererseits reduziert die Verdünnung mit Wasserdampf den Edukt- und Produktpartialdruck, was sich günstig auf die Gleichgewichtslage der Dehydrierung auswirkt. Eine solche Verdünnungswirkung kann auch durch Mitverwendung anderer Inertgase (z. B. N₂, CO₂ etc.) als weitere gasförmige Ausgangsströme bedingt werden. Diesen gegenüber weist Wasserdampf aber, wie bereits gesagt, zusätzlich eine vorteilhafte Wirkung auf die Katalysatorstandzeit in der Reaktionszone A auf.

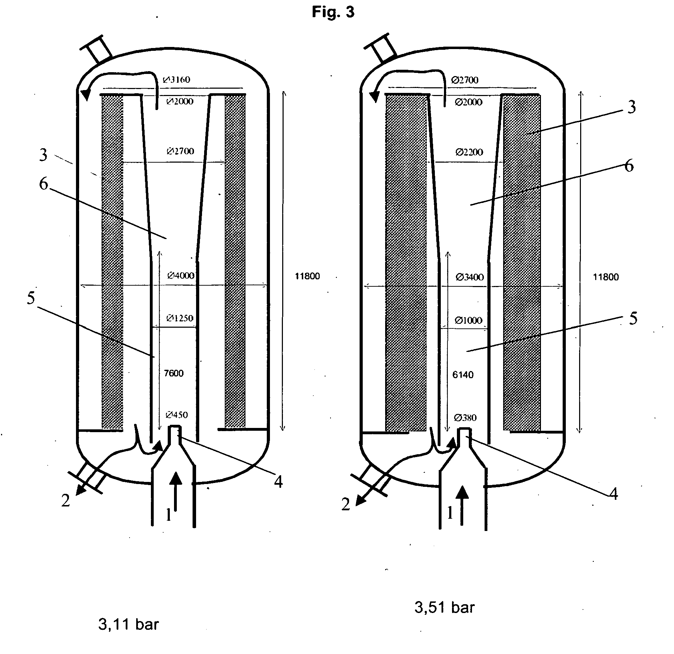

Figur 3 zeigt zwei mögliche Ausgestaltungen einer solchen Reaktionszone A (die in Figur 3 angegebenen Durchmesser haben die Dimension "mm"). Dabei bedeuten die numerischen Adressen:
1 = Treibstrahlgemischstrom
2 = Produktgasgemisch A-Teilstrom 2
3 = Katalysatorfestbett
4 = Treibdüse
5 = Mischrohr
6 = Diffusor

Der Treibstrahlgemischstrom kann dabei z. B. 148 t/h betragen, eine Temperatur von 486°C und einen Druck von 3,11 bar bzw. von 3,51 bar aufweisen. Der Produktgasgemisch A-Teilstrom 2 beträgt in entsprechender Weise gleichfalls 148 t/h und kann z. B. in typischer Weise eine Temperatur von 600°C und einen Druck von 2,3 bar aufweisen. D. h., der Druck auf der Saugseite kann 2,3 bar betragen. Dem entspricht ein Gesamtumwälzstrom von 296 t/h. Als Katalysator können z. B. Stränglinge mit einem Durchmesser von 1,5 mm und einer Länge von in typischer Weise 3 bis 7 mm gemäß Beispiel 4 der DE-A 102 19 879 verwendet werden (Gesamtmenge: z. B. 30 t). Die Schüttdichte derselben im Katalysatorfestbett kann z. B. bei 1200 kg/m³ (locker) bis 1350 kg/m³ (fest) liegen. Unmittelbar vor der Katalysatorschüttung würde dann ein Druck von typisch 2,71 bar vorliegen.

Die wesentlichen Gehalte des Reaktionsgasgemischeingangsstroms A sind typisch:

| | |
|---|---|
| Propen | > 0 bis 25, vielfach 1 bis 10, häufig 2 bis 7 Vol-%; |
| Acrolein | 0 bis 1, vielfach 0 bis 0,5, häufig 0 bis 0,25 Vol.-%; |
| Acrylsäure | 0 bis 0,25, vielfach 0 bis 0,05, häufig 0 bis 0,03 Vol.-%; |
| COₓ | 0 bis 5, vielfach 0 bis 3, häufig 0 bis 2 Vol.-%; |
| Propan | 5 bis 50, vorzugsweise 10 bis 20 Vol.-%; |
| Stickstoff | 30 bis 80, vorzugsweise 50 bis 70 Vol.-%; |
| Sauerstoff | > 0 bis 5, vorzugsweise 1,0 bis 2,0 Vol.-%; |
| H₂O | ≥ 0 bis 20, vorzugsweise 5,0 bis 10,0 Vol.-%; |
| H₂ | 0,5 bis 10, vorzugsweise 1 bis 5 Vol.-%. |

Generell werden jedoch, wie bereits gesagt, möglichst geringe Wasserdampfgehalte im Reaktionsgasgemischeingangsstrom A bevorzugt und angestrebt. Mit zunehmendem in der Reaktionszone A angestrebtem Propanumsatz wächst das Erfordernis eines Beiseins signifikanter Wasserdampfmengen im Reaktionsgasgemischeingangsstrom A um befriedigende Standzeiten der Dehydrierkatalysatoren zu gewährleisten.

Günstig ist es für das erfindungsgemäße Verfahren, wenn das molare Verhältnis von enthaltenem molekularem Wasserstoff zu enthaltenem molekularem Sauerstoff im Reaktionsgasgemischeingangsstrom A etwa 2 : 1 beträgt. Das molare Verhältnis von enthaltenem molekularem Wasserstoff zu enthaltenem Propan ist dabei im Reaktionsgasgemischeingangsstrom A in der Regel gleichzeitig ≤ 5. Das molare Verhältnis von im Reaktionsgasgemischeingangsstrom A enthaltenem Wasserdampf zu darin enthaltenem Propan wird beim erfindungsgemäßen Verfahren vielfach ≥ 0,05 bis 2 bzw. bis 1 betragen.

Mit Vorteil wird beim erfindungsgemäßen Verfahren die Reaktionszone A so gestaltet, dass der Produktgasgemischstrom A nicht umgesetztes Propan und gewünschtes Propylen im molaren Verhältnis Propen zu Propan von 0,2 bzw. 0,3 bis 0,5 (gegebenenfalls bis 0,66) enthält.

Bezogen auf den einmaligen Durchgang des Reaktionsgasgemischeingangsstroms A durch die Reaktionszone A, kann die Reaktionszone A durch gezielten Wärmeaustausch mit außerhalb der Reaktionszone A geführten (fluiden, d.h., flüssigen oder gasförmigen) Wärmeträgern isotherm gestaltet werden. Sie kann mit gleicher Bezugsbasis aber auch adiabat, d.h., im wesentlichen ohne einen solchen gezielten Wärmeaustausch mit außerhalb der Reaktionszone A geführten Wärmeträgern ausgeführt werden. Im letzteren Fall kann die Bruttowärmetönung, bezogen auf den einmaligen Durchgang des der Reaktionszone A zugeführten Reaktionsgasgemischeingangsstroms A durch die Reaktionszone A, durch Ergreifen von in den als Stand der Technik gewürdigten Schriften empfohlenen und im Folgenden noch zu beschreibenden Maßnahmen sowohl endotherm (negativ), oder autotherm (im wesentlichen Null), oder exotherm (positiv) gestaltet werden.

In typischer Weise benötigt die heterogen katalysierte partielle Dehydrierung von Propan zu Propylen vergleichsweise hohe Reaktionstemperaturen. Der dabei erzielbare Umsatz ist normalerweise durch das thermodynamische Gleichgewicht begrenzt. Typische Reaktionstemperaturen betragen 300 bis 800°C bzw. 400 bis 700°C. Pro Molekül an zu Propylen dehydriertem Propan wird dabei ein Molekül Wasserstoff erzeugt.

Hohe Temperaturen und Entfernung des Reaktionsproduktes H₂ begünstigen die Gleichgewichtslage im Sinne des Zielprodukts ebenso wie Pärtialdruckerniedrigung durch inerte Verdünnung.

Grundsätzlich kann die partielle heterogen katalysierte Propandehydrierung in der Reaktionszone A (quasi) adiabat und dabei endotherm durchgeführt werden. Dabei wird der Reaktionsgasgemischeingangsstrom A mit einer Temperatur von 450°C bis 700°C (beziehungsweise von 550 bis 650°C) zum wenigstens einen Katalysatorbett geführt. Beim adiabaten Durchgang durch selbiges (es kommt sowohl ein Wirbelbett als auch ein Festbett in Betracht; ein Katalysatorfestbett ist erfindungsgemäß bevorzugt) wird sich das Reaktionsgasgemisch normalerweise infolge der Wasserstoffverbrennung zunächst erwärmen und dann je nach Umsatz und Verdünnung um etwa 30°C bis 200°C abkühlen. Niedrigere Reaktionstemperaturen ermöglichen längere Standzeiten des verwendeten Katalysatorbetts. Höhere Reaktionstemperaturen fördern erhöhte Umsätze.

Anwendungstechnisch zweckmäßig wird man die heterogen katalysierte Propandehydrierung in der Reaktionszone A als Hordenreaktor verwirklichen.

Dieser enthält räumlich aufeinanderfolgend zweckmäßig mehr als ein die Dehydrierung katalysierendes Katalysatorbett. Die Katalysatorbettenanzahl kann 1 bis 20, zweckmäßig 2 bis 8, aber auch 3 bis 6 betragen. Mit zunehmender Hordenzahl lassen sich zunehmend leichter erhöhte Propanumsätze erreichen. Die Katalysatorbetten sind vorzugsweise radial oder axial hintereinander angeordnet. Anwendungstechnisch zweckmäßig wird in einem solchen Hordenreaktor der Katalysatorfestbetttyp angewendet.

Im einfachsten Fall sind die Katalysatorfestbetten in einem Schachtofenreaktor axial oder in den Ringspalten von zentrisch ineinandergestellten zylindrischen Gitterrosten angeordnet. Es ist jedoch auch möglich, die Ringspalte in Segmenten übereinander anzuordnen und das Gas nach radialem Durchtritt in einem Segment in das nächste darüber oder darunter liegende Segment zu führen.

In zweckmäßiger Weise wird der Reaktionsgasgemischeingangsstrom A auf seinem Weg von einem Katalysatorbett zum nächsten Katalysatorbett, zum Beispiel durch Überleiten über mit heißen Gasen erhitzte Wärmetauscheroberflächen (z. B. Rippen) oder durch Leiten durch mit heißen Brenngasen erhitzte Rohre, im Hordenreaktor einer Zwischenerhitzung unterworfen (Material zweckmäßig Si-haltige Stähle, insbesondere Edelstähle, z. B. vom Typ 1.4841).

Wird der Hordenreaktor im übrigen adiabat betrieben, ist es für wie beschrieben bezogene Propanumsätze ≤ 30 mol-%, vor allem bei Verwendung der in der DE-A 199 37 107 beschriebenen Katalysatoren, insbesondere der beispielhaften Ausführungsformen, ausreichend, den Reaktionsgasgemischeingangsstrom A auf eine Temperatur von 450 bis 550°C vorerhitzt in den Dehydrierreaktor zu führen und innerhalb des Hordenreaktors in diesem Temperaturbereich zu halten. Das heißt, die gesamte Propandehydrierung ist so bei äußerst niederen Temperaturen zu verwirklichen, was sich für die Standzeit der Katalysatorfestbetten als besonders günstig erweist. Für höhere Propanumsätze wird der Reaktionsgasgemischeingangsstrom A zweckmäßig auf höhere Temperaturen vorerhitzt in den Dehydrierreaktor geführt (diese können bis zu 700°C betragen) und innerhalb des Hordenreaktors in diesem erhöhten Temperaturbereich gehalten.

Noch geschickter ist es, die vorstehend geschilderte Zwischenerhitzung auf direktem Weg durchzuführen (autotherme Fahrweise). Dazu wird dem Reaktionsgasgemischstrom normalerweise sowohl hinter dem ersten Katalysatorbett (in Strömungsrichtung betrachtet) als auch zwischen den nachfolgenden Katalysatorbetten in begrenztem Umfang molekularer Sauerstoff zugesetzt. So kann (in der Regel durch die Dehydrierkatalysatoren selbst katalysiert) eine begrenzte Verbrennung des im Reaktionsgasgemisch enthaltenen, im Verlauf der heterogen katalysierten Propandehydrierung gebildeten und/oder dem Reaktionsgasgemisch zugesetztem molekularem Wasserstoff (gegebenenfalls begleitet von einer in geringem Umfang erfolgenden Propanverbrennung) bewirkt werden (es kann auch anwendungstechnisch zweckmäßig sein, im Hordenreaktor Katalysatorbetten einzufügen, die mit Katalysator beschickt sind, der besonders spezifisch (selektiv) die Verbrennung von Wasserstoff katalysiert (als solche Katalysatoren kommen zum Beispiel jene der Schriften US 4,788,371, US 4,886,928, US 5,430,209, US 5,530,171, US 5,527,979 und US 5,563,314 in Betracht; beispielsweise können solche Katalysatorbetten in alternierender Weise zu den Dehydrierkatalysator enthaltenden Betten im Hordenreaktor untergebracht sein). Erfindungsgemäß bevorzugt wird nur zur Bildung des Reaktionsgasgemischeingangsstroms A von außen molekularer Wasserstoff zugesetzt. Die dabei freigesetzte Reaktionswärme ermöglicht so auf quasi autotherme (die Bruttowärmetönung ist im wesentlichen Null) Weise eine nahezu isotherme Betriebsweise der heterogen katalysierten Propandehydrierung. Mit zunehmender gewählter Verweilzeit des Reaktionsgases im Katalysatorbett ist so eine Propandehydrierung bei abnehmender oder im wesentlichen konstanter Temperatur möglich, was besonders lange Katalysatorstandzeiten ermöglicht.

Generell sollte eine wie vorstehend beschriebene Sauerstoffeinspeisung so vorgenommen werden, dass der Sauerstoffgehalt des Reaktionsgasgemisches, bezogen auf die darin enthaltene Menge an molekularem Wasserstoff, 0,5 bis 50 bzw. bis 30, vorzugsweise 10 bis 25 Vol.-% beträgt (diese Relationen sind auch für die entsprechenden Gehalte im Reaktionsgasgemischeingangsstrom A günstig). Als Sauerstoffquelle kommen dabei sowohl reiner molekularer Sauerstoff oder mit Inertgas, zum Beispiel CO, CO₂, N₂ und/oder Edelgase, verdünnter Sauerstoff, insbesondere aber auch Luft (bevorzugt wird ausschließlich Luft als Sauerstoffquelle verwendet), in Betracht. Die resultierenden Verbrennungsgase wirken in der Regel zusätzlich verdünnend und fördern dadurch die heterogen katalysierte Propandehydrierung. Dies gilt insbesondere für den im Rahmen der Verbrennung gebildeten Wasserdampf.

Die Isothermie der heterogen katalysierten Propandehydrierung lässt sich dadurch weiter verbessern, dass man im Hordenreaktor in den Räumen zwischen den Katalysatorbetten geschlossene, vor ihrer Füllung günstigerweise aber nicht notwendigerweise evakuierte, Einbauten (zum Beispiel rohrförmige) anbringt. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Mit Vorteil enthält der Reaktionsgasgemischeingangsstrom A, insbesondere im Fall einer solchen wie beschrieben autothermen Betriebsweise:

| | |
|---|---|
| 15 bis 25 Vol.-% | Propan, |
| 2 bis 6 Vol.-% | Propylen, |
| 5 bis 20 Vol-% | Wasserdampf, |
| 2 bis 10 Vol.-% | molekularer Wasserstoff, |
| 40 bis 75 Vol.-% | molekularer Stickstoff, und |
| > 0 bis 3 Vol.-% | molekularer Sauerstoff. |

Typische Belastungen der Gesamtmenge an Dehydrierkatalysator (Summe über alle Betten) mit Reaktionsgasgemischeingangsstrom A betragen erfindungsgemäß 250 bis 5000 h⁻¹ ( bei Hochlastfahrweise auch bis 40000 h⁻¹), vorzugsweise 10000 bis 25000 Nl/l•h, besonders bevorzugt 15000 bis 20000 Nl/l•h. Die entsprechenden Belastungen mit Propan liegen typisch bei 50 bis 1000 h⁻¹ (bei Hochlastfahrweise auch bis 40000 h⁻¹ ), vorzugsweise bei 2000 bis 5000 Nl/l•h, besonders bevorzugt 3000 bis 4000 Nl/l•h.

Die erfindungsgemäße Verfahrensweise ermöglicht in der Reaktionszone A selbst bei hohen Propanumsätzen Propylenbildungsselektivitäten von 95 mol-% und mehr.

Der der Reaktionszone A (dem Dehydrierreaktor) entnommene Produktgasgemisch A-Teilstrom 2 befindet sich entsprechend den für die heterogen katalysierte Propandehydrierung gewählten Reaktionsbedingungen in der Regel bei einem Druck von 0,3 bis 10 bar, vorzugsweise 1 bis 3 bar, und weist häufig eine Temperatur von 450 bis 650°C bzw. bis 750°C, vielfach eine Temperatur von 500 bis 600°C auf. In der Regel enthält er Propan, Propen, H₂, N₂, H₂O, Methan, Ethan (die letzteren beiden resultieren meist infolge thermischen Zerfalls einer geringen Propanmenge), Ethylen, Buten-1, sonstige Butene wie iso-Buten, andere C₄-Kohlenwasserstoffe wie n-Butan, iso-Butan, Butadien etc., CO und CO₂, im Regelfall aber auch Oxygenate wie Alkohole, Aldehyde und Carbonsäuren (normalerweise mit ≤ 9 C-Atomen). Weiterhin können in geringen Mengen aus dem gasförmigen Ausgangsstrom 2 herrührende Bestandteile enthalten sein.

Während die EP-A 117 146, die DE-A 33 13 573 und die US-A 3,161,670 empfehlen, den Produktgasgemisch A-Teilstrom 2 zur Beschickung des wenigstens einen Oxidationsreaktors zu verwenden, wird es für das erfindungsgemäße Verfahren als vorteilhaft erachtet, aus dem das erforderliche Propylen enthaltenden Produktgasgemisch A-Teilstrom 2 vor seiner Verwendung als Propenquelle für die nachfolgende Propenpartialoxidation wenigstens eine Teilmenge der darin enthaltenen, von Propan und Propylen verschiedenen, Bestandteile abzutrennen. Dabei sollten die Erfordernisse der DE-A 102 11 275 beachtet werden.

Es sei an dieser Stelle zunächst noch festgehalten, dass für die erfindungsgemäße heterogen katalysierte Propandehydrierung grundsätzlich alle im Stand der Technik bekannten Dehydrierkatalysatoren in Betracht kommen. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen. Unter anderem können damit alle Dehydrierkatalysatoren eingesetzt werden, die in der WO 01/96270, der EP-A 731077, der DE-A 10211275, der DE-A 10131297, der WO 99/46039, der US-A 4 788 371, der EP-A-0 705 136, der WO 99/29420, der US-A 4 220 091, der US-A 5 430 220, der US-A 5 877 369, der EP-A-0 117 146, der DE-A 199 37 196, der DE-A 199 37 105 sowie der DE-A 199 37 107 empfohlen werden. Im besonderen können sowohl der Katalysator gemäß Beispiel 1, Beispiel 2, Beispiel 3, und Beispiel 4 der DE-A 199 37 107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Besonders geeignet ist auch der in den Beispielen und Vergleichsbeispielen dieser Schrift eingesetzte Dehydrierkatalysator.

Generell kann es sich bei den Dehydrierkatalysatoren um Katalysatorstränge (Durchmesser typisch 1 bis 10 mm, bevorzugt 1,5 bis 5 mm; Länge typisch 1 bis 20 mm, bevorzugt 3 bis 10 mm), Tabletten (vorzugsweise gleiche Abmessungen wie bei den Strängen) und/oder Katalysatorringe (Außendurchmesser und Länge jeweils typisch 2 bis 30 mm oder bis 10 mm, Wandstärke zweckmäßig 1 bis 10 mm, oder bis 5 mm, oder bis 3 mm) handeln. Zur Durchführung der heterogen katalysierten Dehydrierung im Wirbelbett (bzw. Fließ- oder Wanderbett) wird man entsprechend feinteiligeren Katalysator einsetzen. Das Katalysatorfestbett ist in der Reaktionszone A erfindungsgemäß bevorzugt.

In der Regel sind die Dehydrierkatalysatoren (insbesondere die in dieser Schrift beispielhaft verwendeten sowie die in der DE-A 19937107 empfohlenen (insbesondere die beispielhaften Katalysatoren dieser DE-A) so beschaffen, dass sie sowohl die Dehydrierung von Propan als auch die Verbrennung von Propan und von molekularem Wasserstoff zu katalysieren vermögen. Die Wasserstoffverbrennung verläuft dabei sowohl im Vergleich zur Dehydrierung des Propans als auch im Vergleich zu dessen Verbrennung im Fall einer Konkurrenzsituation an den Katalysatoren sehr viel schneller.

Ferner kommen zur heterogen katalysierten Propandehydrierung prinzipiell alle im Stand der Technik bekannten Reaktortypen und Verfahrensvarianten in Betracht. Beschreibungen solcher Verfahrensvarianten enthalten zum Beispiel alle bezüglich der Dehydrierkatalysatoren zitierten Schriften des Standes der Technik sowie der eingangs dieser Schrift zitierte Stand der Technik.

Eine vergleichsweise ausführliche Beschreibung von erfindungsgemäß geeigneten Dehydrierverfahren enthält auch Catalytica® Studies Division, Oxidative Dehydrogenation and Alternative Dehydrogenation Processes, Study Number 4192 OD, 1993, 430 Ferguson Drive, Mountain View, California, 94043-5272 U.S.A..

Erfindungsgemäß vorteilhaft wird man wenigstens 50 Vol.-%, vorzugsweise wenigstens 75 Vol.-%, besonders bevorzugt wenigstens 90 Vol.-% und ganz besonders bevorzugt wenigstens 95 Vol.-% der im Produktgasgemisch A-Teilstrom 2 enthaltenen, von Propan und Propylen verschiedenen, Bestandteile abtrennen, bevor selbiger als Propenquelle für die Partialoxidation des erfindungsgemäßen Verfahrens eingesetzt wird. Grundsätzlich können dazu alle in den Schriften DE-A 10 2004 032 129, DE-A 10 2005 013 039, DE-A 10 2005 009 891, DE-A 10 2005 010 111, DE-A 10 2005 009 885, DE-A 10 2005 028 798 und DE-A 102 45 585 beschriebenen Abtrennvarianten angewendet werden.

Eine für die erfindungsgemäßen Bedürfnisse zweckmäßige Möglichkeit dazu besteht z.B. darin, den vorzugsweise abgekühlten (vorzugsweise auf Temperaturen von 10 bis 100 bzw. 70°C) Produktgasgemisch A-Teilstrom 2, z. B. bei einem Druck von 0,1 bis 50 bar, vorzugsweise 5 bis 15 bar und einer Temperatur von z. B. 0 bis 100°C, vorzugsweise 20 bis 40°C, mit einem (vorzugsweise hochsiedenden) organischen Lösungsmittel (vorzugsweise ein hydrophobes), in welchem Propan und Propylen (gegenüber den anderen Bestandteilen des Produktgasgemisch A-Teilstroms 2 zweckmäßig bevorzugt) absorbiert werden, in Kontakt zu bringen (z. B. durch einfaches Durchleiten). Durch nachfolgende Desorption, Rektifikation und/oder Strippung mit einem bezüglich der nachfolgenden Propylen-Partialoxidation sich inert verhaltenden und/oder in dieser Partialoxidation als Reaktand benötigten Gas (z. B. Luft oder ein anderes Gemisch aus molekularem Sauerstoff und Inertgas) können das Propan und Propylen im Gemisch in gereinigter Form rückgewonnen und dieses Gemisch für die Partialoxidation als Propylenquelle verwendet werden (vorzugsweise wird wie im Vergleichsbeispiel 1 der deutschen Anmeldung DE-A 10 2004 032 129 beschrieben vorgegangen). Das gegebenenfalls molekularen Wasserstoff enthaltende Abgas einer solchen Absorption kann man z.B. einer Druckwechseladsorptions- und/oder Membrantrennung (z.B. gemäß DE-A 10235419) unterwerfen und dann, den abgetrennten Wasserstoff als Bestandteil des Reaktionsgasgemischeingangsstroms A (als gasförmigen Ausgangsstroms 4) mitverwenden.

Allerdings ist der C3-Kohlenwasserstoffe/C4-Kohlenwasserstoffe Trennfaktor beim vorstehenden Trennverfahren vergleichsweise begrenzt und für die in der DE-A 10245585 beschriebenen Bedürfnisse häufig nicht ausreichend.

Als Alternative für den beschriebenen Trennschritt via Absorption ist für die erfindungsgemäßen Zwecke daher häufig eine Druckwechseladsorption oder eine Druckrektifikation bevorzugt.

Als Absorptionsmittel für die vorstehend beschriebene absorptive Abtrennung eignen sich grundsätzlich alle Absorptionsmittel, die in der Lage sind, Propan und Propylen zu absorbieren. Bei dem Absorptionsmittel handelt es sich vorzugsweise um ein organisches Lösungsmittel, das vorzugsweise hydrophob und/oder hochsiedend ist. Vorteilhafterweise hat dieses Lösungsmittel einen Siedepunkt (bei einem Normaldruck von 1 atm) von mindestens 120°C, bevorzugt von mindestens 180°C, vorzugsweise von 200 bis 350°C, insbesondere von 250 bis 300°C, stärker bevorzugt von 260 bis 290°C. Zweckmäßigerweise liegt der Flammpunkt (bei einem Normaldruck von 1 bar) über 110°C. Allgemein eignen sich als Absorptionsmittel relativ unpolare organische Lösungsmittel, wie zum Beispiel, aliphatische Kohlenwasserstoffe, die vorzugsweise keine nach außen wirkende polare Gruppe enthalten, aber auch aromatische Kohlenwasserstoffe. Allgemein ist es erwünscht, dass das Absorptionsmittel einen möglichst hohen Siedepunkt bei gleichzeitig möglichst hoher Löslichkeit für Propan und Propylen hat. Beispielhaft als Absorptionsmittel genannt seien aliphatische Kohlenwasserstoffe, zum Beispiel C₈-C₂₀-Alkane oder -Alkene, oder aromatische Kohlenwasserstoffe, zum Beispiel Mittelölfraktionen aus der Paraffindestillation oder Ether mit sperrigen (sterisch anspruchvollen) Gruppen am O-Atom, oder Gemische davon, wobei diesen ein polares Lösungsmittel, wie zum Beispiel das in der DE-A 43 08 087 offenbarte 1,2-Dimethylphthalat zugesetzt sein kann. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkene, zum Beispiel 4-Methyl-4'-benzyl-diphenylmethan und dessen Isomere 2-Methyl-2'-benzyl-diphenyl-methan, 2-Methyl-4'-benzyldiphenylmethan und 4-Methyl-2'-benzyl-diphenylmethan und Gemische solcher Isomerer. Ein geeignetes Absorptionsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether; beispielsweise das im Handel erhältliche Diphyl® (z. B. von der Bayer Aktiengesellschaft bezogenes). Häufig enthält dieses Lösungsmittelgemisch ein Lösungsmittel wie Dimethylphthalat in einer Menge von 0,1 bis 25 Gew.-%, bezogen auf das gesamte Lösungsmittelgemisch, zugesetzt. Besonders geeignete Absorptionsmittel sind auch Octane, Nonane, Decane, Undecane, Dodecane, Tridecane, Tetradecane, Pentadecane, Hexadecane, Heptadecane und Octadecane, wobei sich insbesondere Tetradecane als besonders geeignet erwiesen haben. Günstig ist es, wenn das verwendete Absorptionsmittel einerseits den oben genannten Siedepunkt erfüllt, andererseits gleichzeitig aber ein nicht zu hohes Molekulargewicht aufweist. Mit Vorteil beträgt das Molekulargewicht des Absorptionsmittels ≤ 300 g/mol. Geeignet sind auch die in der DE-A 33 13 573 beschriebenen Paraffinöle mit 8 bis 16 Kohlenstoffatomen. Beispiele für geeignete Handelsprodukte sind von der Firma Haltermann vertriebene Produkte wie Halpasole i, wie Halpasol 250/340 i und Halpasol 250/275 i, sowie Druckfarbenöle unter den Bezeichnungen PKWF und Printosol. Bevorzugt sind an Aromaten freie Handelsprodukte, z. B. solche des Typs PKWFaf. Falls sie einen geringen Restaromatengehalt enthalten, kann dieser vorab des beschriebenen Einsatzes vorteilhaft rektifikativ und/oder adsorptiv gemindert und auf Werte signifikant unter 1000 gew.ppm gedrückt werden. Weitere geeignete Handelsprodukte sind n-Paraffin (C₁₃-C₁₇) oder Mihagol®5 der Erdöl-Raffinerie-Emsland GmbH, LINPAR®14-17 der Firma CONDEA Augusta S.p.A. (Italien) oder der SASOL Italy S.p.A., Normal parrafins (heavy) C₁₄-C₁₈ der Firma SLONAFT in der Slowakei.

Die Gehalte (angegeben in Flächenprozenten der gaschromatographischen Analyse) der vorgenannten Produkte an linearen Kohlenwasserstoffen betragen in typischer Weise:
Summe C₉ bis C₁₃: weniger als 1 %; C₁₄: 30 bis 40 %; C₁₅: 20 bis 33 %; C₁₆: 18 bis 26 %; C₁₇: bis zu 18 %; C_{≥18}: < 2 %.
Eine typische Zusammensetzung des Produktes der Fa. SASOL ist: C₁₃: 0,48 %; C₁₄: 39,8 %; C₁₅: 20,8 %; C₁₆: 18,9 %; C₁₇: 17,3 %; C₁₈: 0,91 %; C₁₉: 0,21 %.
Eine typische Zusammensetzung des Produktes der Fa. Haltermann ist: C₁₃: 0,58 %; C₁₄: 33,4 %; C₁₅: 32,8 %; C₁₆: 25,5 %; C₁₇: 6,8 %; C_{≥18}: < 0,2 %.

Im kontinuierlichen Betrieb wird sich die Zusammensetzung des Absorptionsmittels verfahrensbedingt entsprechend ändern.

Die Durchführung der Absorption unterliegt keinen besonderen Beschränkungen. Es können alle dem Fachmann gängigen Verfahren und Bedingungen eingesetzt werden. Vorzugsweise wird der Produktgasgemisch A-Teilstrom 2 bei einem Druck von 1 bis 50 bar, bevorzugt 2 bis 20 bar, stärker bevorzugt 5 bis 15 bar, und einer Temperatur von 0 bis 100°C, insbesondere von 20 bis 50 bzw. 40°C, mit dem Absorptionsmittel in Kontakt gebracht. Die Absorption kann sowohl in Kolonnen als auch in Quenchapparaten vorgenommen werden. Dabei kann im Gleichstrom oder im Gegenstrom (ist bevorzugt) gearbeitet werden. Geeignete Absorptionskolonnen sind zum Beispiel Bodenkolonnen (mit Glocken- und/oder Siebböden), Kolonnen mit strukturierten Packungen (zum Beispiel Blechpackungen mit einer spezifischen Oberfläche von 100 bis 1000, oder bis 750 m²/m³, zum Beispiel Mellapak® 250 Y) und Füllkörperkolonnen (zum Beispiel mit Raschig-Füllkörpern gefüllte). Es können aber auch Riesel- und Sprühtürme, Graphitblockabsorber, Oberflächenabsorber wie Dickschicht- und Dünnschichtabsorber sowie Tellerwäscher, Kreuzschleierwäscher und Rotationswäscher verwendet werden. Zudem kann es günstig sein, die Absorption in einer Blasensäule mit und ohne Einbauten stattfinden zu lassen.

Die Abtrennung des Propans und Propylens von dem Absorptionsmittel kann durch Strippung, Entspannungsverdampfung (Flashen) und/oder Destillation erfolgen.

Die Abtrennung des Propans und Propylens von dem Absorptionsmittel erfolgt vorzugsweise durch Strippen und/oder Desorption. Die Desorption kann in üblicher Weise über eine Druck- und/oder Temperaturänderung durchgeführt werden, vorzugsweise bei einem Druck von 0,1 1 bis 10 bar, insbesondere 1 bis 5 bar, stärker bevorzugt 1 bis 3 bar, und einer Temperatur von 0 bis 200°C, insbesondere 20 bis 100°C, stärker bevorzugt 30 bis 70°C, besonders bevorzugt 30 bis 50°C. Ein für die Strippung geeignetes Gas ist beispielsweise Wasserdampf, bevorzugt sind jedoch insbesondere Sauerstoff-/Stickstoff-Mischungen, beispielsweise Luft. Bei der Verwendung von Luft beziehungsweise Sauerstoff-/Stickstoff-Mischungen, bei denen der Sauerstoffgehalt über 10 Vol-% liegt, kann es sinnvoll sein, vor und/oder während des Strippprozesses ein Gas zuzusetzen, das den Explosionsbereich reduziert. Besonders geeignet dafür sind Gase mit einer spezifischen Wärmekapazität ≥ 29 J/mol•K bei 20°C, wie zum Beispiel Methan, Ethan, Propan (bevorzugt), Propen, Benzol, Methanol, Ethanol, sowie Ammoniak, Kohlendioxid, und Wasser. C4-Kohlenwasserstoffe sind erfindungsgemäß bevorzugt als solche Zusätze jedoch zu vermeiden. Besonders geeignet für die Strippung sind auch Blasensäulen mit und ohne Einbauten.

Die Abtrennung des Propans und Propylens von dem Absorptionsmittel kann auch über eine Destillation bzw. Rektifikation erfolgen, wobei die dem Fachmann geläufigen Kolonnen mit Packungen, Füllkörpern oder entsprechenden Einbauten verwendet werden können. Bevorzugte Bedingungen bei der Destillation bzw. Rektifikation sind ein Druck von 0,01 bis 5 bar, insbesondere 0,1 bis 4 bar, stärker bevorzugt 1 bis 3 bar, und eine Temperatur (im Sumpf) von 50 bis 300°C, insbesondere 150 bis 250°C.

Eine durch Strippen aus dem Absorptionsmittel gewonnene für die Reaktionszone B des erfindungsgemäßen Verfahrens prinzipiell geeignete Propylenquelle kann vor ihrer Verwendung zur Beschickung der Partialoxidation noch einer weiteren Verfahrensstufe zugeführt werden, um z. B. die Verluste an mitgestripptem Absorptionsmittel zu reduzieren (z. B. Abscheidung in Demistern und/oder Tiefenfiltern) und die erfindungsgemäß durchzuführende Partialoxidation so gleichzeitig vor Absorptionsmittel zu schützen oder um die Trennwirkung zwischen C3-/C4-Kohlenwasserstoffen weiter zu verbessern. Eine solche Abtrennung des Absorptionsmittels kann nach allen dem Fachmann bekannten Verfahrensschritten erfolgen. Eine im Rahmen des erfindungsgemäßen Verfahrens bevorzugte Ausführungsform einer solchen Abtrennung ist z. B. das Quenchen des Ausgangsstromes aus der Strippvorrichtung mit Wasser. In diesem Fall wird das Absorptionsmittel aus diesem beladenen Ausgangsstrom mit Wasser herausgewaschen und der Ausgangsstrom gleichzeitig mit Wasser beladen (geringe Wassermengen wirken sich förderlich auf die Aktivität der Katalysatoren für die erfindungsgemäße Partialoxidation aus). Diese Wäsche beziehungsweise das Quenchen kann z. B. am Kopf einer Desorptionskolonne über einem Flüssigkeits-Fangboden durch Gegensprühen von Wasser oder in einem eigenen Apparat erfolgen.

Zur Unterstützung des Trenneffektes können im Quenchraum die Quenchoberfläche vergößernde Einbauten installiert werden, wie sie dem Fachmann von Rektifikationen, Absorptionen und Desorptionen her bekannt sind.

Wasser ist insofern ein bevorzugtes Waschmittel, da es in der nachfolgenden Partialoxidation normalerweise nicht wesentlich stört. Nachdem das Wasser das Absorptionsmittel aus dem mit Propan und Propylen beladenen Ausgangstrom herausgewaschen hat, kann das Wasser/Absorptionsmittel-Gemisch einer Phasentrennung zugeführt und der behandelte, volumenarme, Ausgangstrom, unmittelbar der erfindungsgemäß durchzuführenden Partialoxidation zugeführt werden.

In für das erfindungsgemäße Verfahren vorteilhafter Weise lassen sich insbesondere dann, wenn das Propylen/Propan-Gemisch aus dem Absorbat mittels Luft freigestrippt wird, in der Regel unmittelbar für die Partialoxidation verwendbare Reaktionsgasausgangsgemische gewinnen. Für den Fall, dass deren Propangehalt erfindungsgemäß noch nicht befriedigen sollte, kann ihnen vor ihrer Verwendung für die erfindungsgemäß durchzuführende Partialoxidation des enthaltenen Propylens noch Frischpropan zugesetzt werden. Über das Restgas (den gasförmigen Ausgangsstrom 2) wird selbiges dann zweckmäßig in die heterogen katalysierte Dehydrierung (als Bestandteil des Reaktionsgasgemischeingangsstroms A) rückgeführt werden. Um die entsprechende Propanmenge kann dann die Zufuhr an Frischpropan über den gasförmigen Ausgangsstrom 3 gemindert werden. Im Extremfall kann die in der heterogen katalysierten Propandehydrierung erforderliche Zufuhr an Frischpropan dann vollständig entfallen, wenn diese Zufuhr an Frischpropan vor der Durchführung der Partialoxidation des Propylens vollständig ins diesbezügliche Reaktionsgasausgangsgemisch hinein erfolgt, von wo aus es dann als verbleibender Bestandteil im Restgas erst nach Durchlaufen der erfindungsgemäß durchzuführenden Partialoxidation dem Reaktionsgasgemischeingangsstrom A für die heterogen katalysierte Propandehydrierung zugeführt wird. Gegebenenfalls kann eine Frischpropanzufuhr auch in eine gegebenenfalls zwischen heterogen katalysierte Dehydrierung und Propylenpartialoxidation befindliche C₃-Abtrennung hinein (z. B. als Stripgas) erfolgen.

Handelt es sich um eine zweistufige Partialoxidation von Propylen zu Acrylsäure, kann ein Teil oder auch die vollständige Zufuhr an Frischpropan auch in das Reaktionsgasausgangsgemisch für die zweite Stufe der Partialoxidation hinein erfolgen (allerdings ist dieses Reaktionsgasausgangsgemisch manchmal bereits dann nicht explosiv, wenn diese Qualifizierung schon auf das Reaktionsgasausgangsgemisch für die erste Stufe der Partialoxidation zutraf). Dies ist vor allem deshalb vorteilhaft, weil die unerwünschte Nebenreaktion von Propan zu Propionaldehyd und/oder Propionsäure vor allem von der ersten Partialoxidationsstufe (Propylen → Acrolein) unter deren Bedingungen ausgeht. Vorteilhaft ist es auch, eine Frischpropanzufuhr auf die erste und auf die zweite Partialoxidationsstufe im wesentlichen gleichmäßig zu verteilen.

Durch diese Möglichkeit der Zufuhr von Frischpropan ins Reaktionsgasausgangsgemisch für die Partialoxidationsstufen hinein, lässt sich die Zusammensetzung dieser Reaktionsgasausgangsgemische zielsicher nicht explosiv gestalten. Gegebenenfalls kann auch eine Teilmenge an Restgas zu diesem Zweck direkt in die Propylen- und/oder Acroleinpartialoxidation rückgeführt werden. Bei Bedarf kann auch ein Gemisch aus Frischpropan und Restgas für diesen Zweck verwendet werden. Entscheidend für die Beantwortung der Frage, ob das Reaktionsgasausgangsgemisch für eine Partialoxidationsstufe explosiv ist oder nicht, ist, ob sich in dem unter bestimmten Ausgangsbedingungen (Druck, Temperatur) befindlichen Reaktionsgasausgangsgemisch eine durch eine örtliche Zündquelle (z. B. glühender Platindraht) eingeleitete Verbrennung (Entzündung, Explosion) ausbreitet oder nicht (vgl. DIN 51649 und die Untersuchungsbeschreibung in der WO 04/007405). Erfolgt eine Ausbreitung, soll das Gemisch hier als explosiv bezeichnet werden. Erfolgt keine Ausbreitung, wird das Gemisch in dieser Schrift als nicht explosiv eingeordnet. Ist das Reaktionsgasausgangsgemisch nicht explosiv, gilt dies auch für die im Verlauf der erfindungsgemäßen Partialoxidation des Propylens gebildeten Reaktionsgasgemische (vgl. WO 04/007405).

In der Regel wird beim erfindungsgemäßen Verfahren jedoch nur der Reaktionsgasgemischeingangsstrom A Frischpropan zugesetzt enthalten.

Vorliegende Erfindung betrifft aber auch Verfahrensausgestaltungen, bei denen man das für das Verfahren benötigte Frischpropan höchstens teilweise (z. B. nur zu 75 %, oder nur zu 50 %, oder nur zu 25 %) dem Reaktionsgasgemischeingangsstrom A und wenigstens teilweise (in der Regel die Restmenge, gegebenenfalls die Gesamtmenge) dem bzw. den Reaktionsgasgemischausgangsgasen der Partialoxidation zuführt. Im übrigen kann wie in der WO 01/96170 beschrieben verfahren werden, die einen integralen Bestandteil dieser Anmeldung bildet.

In an sich bekannter Weise läuft die heterogen katalysierte Gasphasen-Partialoxidation von Propylen zu Acrylsäure mit molekularem Sauerstoff grundsätzlich in zwei längs der Reaktionskoordinate aufeinanderfolgenden Schritten ab, von denen der erste zum Acrolein und der zweite von Acrolein zu Acrylsäure führt.

Dieser Reaktionsablauf in zwei zeitlich aufeinanderfolgenden Schritten eröffnet in an sich bekannter Weise die Möglichkeit, das erfindungsgemäße Verfahren auf der Stufe des Acrolein (der Stufe überwiegender Acroleinbildung) abzubrechen und die Zielproduktabtrennung auf dieser Stufe vorzunehmen, oder das erfindungsgemäße Verfahren bis zur überwiegenden Acrylsäurebildung fortzuführen und die Zielproduktabtrennung erst dann vorzunehmen.

Führt man das erfindungsgemäße Verfahren bis zur überwiegenden Acrylsäurebildung, ist es erfindungsgemäß vorteilhaft, das Verfahren zweistufig, d. h. in zwei hintereinander angeordneten Oxidationsstufen auszuführen, wobei zweckmäßigerweise in jeder der beiden Oxidationsstufen das zu verwendende Katalysatorfestbett und vorzugsweise auch die sonstigen Reaktionsbedingungen, wie z. B. die Temperatur des Katalysatorfestbetts, in optimierender Weise angepasst werden.

Zwar vermögen die für die Katalysatoren der ersten Oxidationsstufe (Propylen -> Acrolein) als Aktivmassen besonders geeigneten, die Elemente Mo, Fe, Bi enthaltenden Multimetalloxide, in gewissem Umfang auch die zweite Oxidationsstufe (Acrolein -> Acrylsäure) zu katalysieren, doch werden für die zweite Oxidationsstufe normalerweise Katalysatoren bevorzugt, deren Aktivmasse wenigstens ein die Elemente Mo und V enthaltendes Multimetalloxid ist.

Damit eignet sich das erfindungsgemäß durchzuführende Verfahren der heterogen katalysierten Partialoxidation von Propylen an Katalysatorfestbetten, deren Katalysatoren als Aktivmasse wenigstens ein die Elemente Mo, Fe und Bi enthaltendes Multimetalloxid aufweisen, insbesondere als einstufiges Verfahren zur Herstellung von Acrolein (sowie gegebenenfalls Acrylsäure) oder als erste Reaktionsstufe zur zweistufigen Herstellung von Acrylsäure.

Die Realisierung der einstufigen heterogen katalysierten Partialoxidation von Propylen zu Acrolein sowie gegebenenfalls Acrylsäure bzw. der zweistufigen heterogen katalysierten Partialoxidation von Propylen zu Acrylsäure unter Verwendung eines erfindungsgemäß erzeugten Reaktionsgasausgangsgemischs kann dabei im einzelnen wie in den Schriften EP-A 70 07 14 (erste Reaktionsstufe; wie dort beschrieben, aber auch in entsprechender Gegenstromfahrweise von Salzbad und Reaktionsgasausgangsgemisch über den Rohrbündelreaktor), EP-A 70 08 93 (zweite Reaktionsstufe; wie dort beschrieben, aber auch in entsprechender Gegenstromfahrweise), WO 04/085369 (insbesondere diese Schrift wird als integraler Bestandteil dieser Schrift betrachtet) (als zweistufiges Verfahren), WO 04/85363, DE-A 103 13 212 (erste Reaktionsstufe), EP-A 1 159 248 (als zweistufiges Verfahren), EP-A 1 159 246 (zweite Reaktionsstufe), EP-A 1 159 247 (als zweistufiges Verfahren), DE-A 199 48 248 (als zweistufiges Verfahren), DE-A 101 01 695 (einstufig oder zweistufig), WO 04/085368 (als zweistufiges Verfahren), DE-A 10 2004 021 764 (zweistufig), WO 04/085362 (erste Reaktionsstufe), WO 04/085370 (zweite Reaktionsstufe), WO 04/085365 (zweite Reaktionsstufe), WO 04/085367 (zweistufig), EP-A 990 636, EP-A 1 007 007 und EP-A 1 106 598 beschrieben durchgeführt werden.

Dies gilt insbesondere für alle in diesen Schriften enthaltenen Ausführungsbeispiele. Sie können wie in diesen Schriften beschrieben durchgeführt werden, jedoch mit dem Unterschied, dass als Reaktionsgasausgangsgemisch für die erste Reaktionsstufe (Propylen zu Acrolein) ein erfindungsgemäß erzeugtes Reaktionsgasausgangsgemisch 2 eingesetzt wird. Die übrigen Parameter betreffend wird wie in den Ausführungsbeispielen der genannten Schriften verfahren (insbesondere die Katalysatorfestbetten und Reaktandenbelastung der Katalysatorfestbetten betreffend). Wird in den vorgenannten Ausführungsbeispielen des Standes der Technik zweistufig verfahren und erfolgt zwischen den beiden Reaktionsstufen eine Sekundärsauerstoff(Sekundärluft)einspeisung, so wird diese in entsprechender Weise vorgenommen, in ihrer Menge jedoch dahingehend angepasst, dass das molare Verhältnis von molekularem Sauerstoff zu Acrolein im Beschickungsgemisch der zweiten Reaktionsstufe demjenigen in den Ausführungsbeispielen der genannten Schriften entspricht. Ein erfindungsgemäßes Reaktionsgasausgangsgemisch für die heterogen katalysierte partielle Gasphasenoxidation ist z. B. in einfacher Weise dadurch erhältlich, dass man dem Produktgasgemisch A-Teilstrom 2 oder dem Produktgasgemischstrom A' soviel molekularen Sauerstoff zusetzt, wie für die Partialoxidation benötigt wird. Diese Zufuhr kann als reiner molekularer Sauerstoff oder auch als ein Gemisch aus molekularem Sauerstoff und aus Inertgas (oder auch nur im Beisein von Inertgas) erfolgen. Als ein solches Gemisch wird erfindungsgemäß Luft bevorzugt. Erfindungsgemäß wesentlich ist, dass diese Sauerstoffzufuhr so erfolgt, dass das Produktgasgemisch B noch nicht umgesetzten molekularen Sauerstoff enthält.

In der Regel wird das molare Verhältnis von im Reaktionsgasausgangsgemisch für die Partialoxidation enthaltenem molekularem Sauerstoff zu im Reaktionsgasausgangsgemisch für die Partialoxidation enthaltenem Propylen ≥ 1 und ≤ 3 betragen.

Für die jeweilige Reaktionsstufe besonders geeignete Multimetalloxidkatalysatoren sind vielfach vorbeschrieben und dem Fachmann wohlbekannt. Beispielsweise verweist die EP-A 25 34 09 auf Seite 5 auf entsprechende US-Patente.

Günstige Katalysatoren für die jeweilige Oxidationsstufe offenbaren auch die DE-A 4 431 957, die DE-A 10 2004 025 445 und die DE-A 4 431 949. Dieses gilt insbesondere für jene der allgemeinen Formel I in den beiden vorgenannten älteren Schriften. Besonders vorteilhafte Katalysatoren für die jeweilige Oxidationsstufe offenbaren die Schriften DE-A 103 25 488, DE-A 103 25 487, DE-A 103 53 954, DE-A 103 44 149, DE-A 103 51 269, DE-A 103 50 812 und DE-A 103 50 822.

Für die erfindungsgemäße Reaktionsstufe der heterogen katalysierten Gasphasen-Partialoxidation von Propylen zu Acrolein oder Acrylsäure oder deren Gemisch, kommen prinzipiell alle Mo, Bi und Fe enthaltenden Multimetalloxidmassen als Aktivmasse in Betracht.

Dies sind insbesondere die Multimetalloxidaktivmassen der allgemeinen Formel I der DE-A 199 55 176, die Multimetalloxidaktivmassen der allgemeinen Formel I der DE-A 199 48 523, die Multimetalloxidaktivmassen der allgemeinen Formeln I, II und III der DE-A 101 01 695, die Multimetalloxidaktivmassen der allgemeinen Formeln I, II und III der DE-A 199 48 248 und die Multimetalloxidaktivmassen der allgemeinen Formeln I, II und III der DE-A 199 55 168 sowie die in der EP-A 7 00 714 genannten Multimetalloxidaktivmassen.

Ferner eignen sich für diese Reaktionsstufe die Mo, Bi und Fe enthaltenden Multimetalloxidkatalysatoren die in den Schriften Research Disclosure Nr. 497012 vom 29.08.2005, DE-A 100 46 957, DE-A 100 63 162, DE-C 3 338 380, DE-A 199 02 562, EP-A 15 565, DE-C 2 380 765, EP-A 8 074 65, EP-A 27 93 74, DE-A 330 00 44, EP-A 575897, US-A 4438217, DE-A 19855913, WO 98/24746, DE-A 197 46 210 (diejenigen der allgemeinen Formel II), JP-A 91/294239, EP-A 293 224 und EP-A 700 714 offenbart werden. Dies gilt insbesondere für die beispielhaften Ausführungsformen in diesen Schriften, unter denen jene der EP-A 15565, der EP-A 575897, der DE-A 197 46 210 und der DE-A 198 55 913 besonders bevorzugt werden. Besonders hervorzuheben sind in diesem Zusammenhang ein Katalysator gemäß Beispiel 1c aus der EP-A 15 565 sowie ein in entsprechender Weise herzustellender Katalysator, dessen Aktivmasse jedoch die Zusammensetzung Mo₁₂Ni_{6.5}Zn₂Fe₂Bi₁P_{0,0065}K_{0,006}Oₓ•10SiO₂ aufweist. Ferner sind hervorzuheben das Beispiel mit der laufenden Nr. 3 aus der DE-A 198 55 913 (Stöchiometrie: Mo₁₂Co₇Fe₃Bi_{0,6}K_{0,08}Si_{1,6}Oₓ) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 197 46 210. Ferner wären die Multimetalloxid-Katalysatoren der US-A 4,438,217 zu nennen. Letzteres gilt insbesondere dann, wenn diese Hohlzylinder eine Geometrie 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Weitere mögliche Katalysatorgeometrien sind in diesem Zusammenhang Stränge (z. B. 7,7 mm Länge und 7 mm Durchmesser; oder 6,4 mm Länge und 5,7 mm Durchmesser).

Eine Vielzahl der für den Schritt von Propylen zu Acrolein und gegebenenfalls Acrylsäure geeigneten Multimetalloxidaktivmassen lässt sich unter der allgemeinen Formel IV,

Mo₁₂BiₐFe_{b}X¹_{c}X²_{d}X³ₑX⁴_{f}Oₙ (IV),

in der die Variablen nachfolgende Bedeutung aufweisen:

| | |
|---|---|
| X¹ = | Nickel und/oder Kobalt, |
| X² = | Thallium, ein Alkalimetall und/oder ein Erdalkalimetall, |
| X³ = | Zink, Phosphor, Arsen, Bor, Antimon, Zinn, Cer, Blei und/oder Wolfram, |
| X⁴ | = Silicium, Aluminium, Titan und/oder Zirkonium, |
| | |
| a= | 0,5 bis 5, |
| b= | 0,01 bis 5, vorzugsweise 2 bis 4, |
| c= | 0 bis 10, vorzugsweise 3 bis 10, |
| d= | 0 bis 2, vorzugsweise 0,02 bis 2, |
| e= | 0 bis 8, vorzugsweise 0 bis 5, |
| f= | 0 bis 10 und |
| n= | eine Zahl, die durch die Wertigkeit und Häufigkeit der von Sauerstoff verschiedenen Elemente in IV bestimmt wird, |

subsumieren.

Sie sind in an sich bekannter Weise erhältlich (siehe z. B. die DE-A 4 023 239) und werden üblicherweise in Substanz zu Kugeln, Ringen oder Zylindern geformt oder auch in Gestalt von Schalenkatalysatoren, d. h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel IV in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemisch aus Inertgas, NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie NH₄OH (NH₄)₂CO₃, NH₄NO₃, NH₄CHO₂, CH₃COOH, NH₄CH₃CO₂ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die Multimetalloxidaktivmassen der allgemeinen Formel IV können für den Schritt "Propylen → Acrolein (und gegebenenfalls Acrylsäure)" sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Anstelle von Graphit kann aber auch hexagonales Bornitrid als Hilfsmittel bei der Formgebung verwendet werden, wie es die DE-A 10 2005 037 678 empfiehlt. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 29 09 671, der EP-A 293 859 oder aus der EP-A 714 700 bekannt ist. Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für den Schritt vom Propylen zum Acrolein (sowie gegebenenfalls Acrylsäure) zu verwendende Multimetalloxidaktivmassen sind ferner Massen der allgemeinen Formel V,

[Y¹_{a'}Y²_{b'}O_{x'}]ₚ[Y³_{c'}Y⁴_{d'}Y⁵_{e'}Y⁶_{f'}Y⁷_{g'}Y²_{h'}O_{y'}]_{q} (V),

in der die Variablen folgende Bedeutung haben:

| | |
|---|---|
| Y¹ = | nur Wismut oder Wismut und wenigstens eines der Elemente Tellur, Antimon, Zinn und Kupfer, |
| Y² = | Molybdän, oder Wolfram, oder Molybdän und Wolfram, |
| Y³ = | ein Alkalimetall, Thallium und/oder Samarium, |
| Y⁴ = | ein Erdalkalimetall, Nickel, Kobalt, Kupfer, Mangan, Zink, Zinn, Cadmium und/oder Quecksilber, |
| Y⁵ = | Eisen oder Eisen und wenigstens eines der Elemente Chrom und Cer, |
| Y⁶= | Phosphor, Arsen, Bor und/oder Antimon, |
| Y⁷ = | ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran, |
| | |
| a' = | 0,01 bis 8, |
| b' = | 0,1 bis 30, |
| c' = | 0 bis 4, |
| d' = | 0 bis 20, |
| e' > | 0 bis 20, |
| f = | 0 bis 6, |
| g' = | 0 bis 15, |
| h' = | 8 bis 16, |
| x',y'= | Zahlen, die durch die Wertigkeit und Häufigkeit der von Sauerstoff verschiedenen Elemente in V bestimmt werden und |
| p,q = | Zahlen, deren Verhältnis p/q 0,1 bis 10 beträgt, |

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹_{a'}Y²_{b'}O_{x'}, deren Größtdurchmesser (längste durch den Schwerpunkt des Bereichs gehende direkte Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte erfindungsgemäß geeignete Multimetalloxidmassen V sind solche, in denen Y¹ nur Wismut ist.

Unter diesen werden wiederum jene bevorzugt, die der allgemeinen Formel VI,

[Bi_{a"}Z²_{b"}O_{x"}]_{p"} [Z²₁₂Z³_{c"}Z⁴_{d"}Fe_{e"}Z⁵_{f"}Z⁶_{g"}Z⁷_{h"}O_{y"}]_{q"} (VI),

in der die Variablen folgende Bedeutung haben:

| | |
|---|---|
| Z² = | Molybdän, oder Wolfram, oder Molybdän und Wolfram, |
| Z³ = | Nickel und/oder Kobalt, |
| Z⁴ = | Thallium, ein Alkalimetall und/oder ein Erdalkalimetall, |
| Z⁵ = | Phosphor, Arsen, Bor, Antimon, Zinn, Cer und/oder Blei, |
| Z⁶ = | Silicium, Aluminium, Titan und/oder Zirkonium, |
| Z⁷ = | Kupfer, Silber und/oder Gold, |
| | |
| a" = | 0,1 bis 1, |
| b" = | 0,2 bis 2, |
| c" = | 3 bis 10, |
| d" = | 0,02 bis 2, |
| e" = | 0,01 bis 5, vorzugsweise 0,1 bis 3, |
| f' = | 0 bis 5, |
| g" = | 0 bis 10, |
| h" | = 0 bis 1, |
| x",y"= | Zahlen, die durch die Wertigkeit und Häufigkeit der von Sauerstoff verschiedenen Element in VI bestimmt werden, |
| p",q"= | Zahlen, deren Verhältnis p"/q" 0,1 bis 5, vorzugsweise 0,5 bis 2 beträgt, |

entsprechen, wobei diejenigen Massen VI ganz besonders bevorzugt werden, in denen Z²_{b"} = (Wolfram)_{b"} und Z²₁₂ = (Molybdän)₁₂ ist.

Ferner ist es von Vorteil, wenn wenigstens 25 mol-% (bevorzugt wenigstens 50 mol-% und besonders bevorzugt 100 mol-%) des gesamten Anteils [Y¹_{a'}Y²_{b'}O_{x'}]ₚ ([Bi_{a"}Z²_{b"}O_{x"}]_{p"}) der erfindungsgemäß geeigneten Multimetalloxidmassen V (Multimetalloxidmassen VI) in den erfindungsgemäß geeigneten Multimetalloxidmassen V (Multimetalloxidmassen VI) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y¹_{a'}Y²_{b'}O_{x'} [Bi_{a"}Z²_{b"}O_{x"}] vorliegen, deren Größtdurchmesser im Bereich 1 nm bis 100 µm liegt.

Hinsichtlich der Formgebung gilt bezüglich Multimetalloxidmassen V-Katalysatoren das bei den Multimetalloxidmassen IV-Katalysatoren Gesagte.

Die Herstellung von Multimetalloxidmassen V-Aktivmassen ist z. B. in der EP-A 575 897 sowie in der DE-A 198 55 913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende und/oder das Gasgemisch aufheizende Vorschüttung in Betracht.

Für den zweiten Schritt(die zweite Reaktionsstufe), die heterogen katalysierte Gasphasen-Partialoxidation von Acrolein zu Acrylsäure, kommen, wie bereits gesagt, prinzipiell alle Mo und V enthaltenden Multimetalloxidmassen als Aktivmassen für die benötigten Katalysatoren in Betracht, z. B. jene der DE-A 100 46 928.

Eine Vielzahl derselben, z. B. diejenigen der DE-A 198 15 281, lässt sich unter der allgemeinen Formel VII,

Mo₁₂VₐX¹_{b}X²_{c}X³_{d}X⁴ₑX⁵_{f}X⁶_{g}Oₙ (VII),

in der die Variablen folgende Bedeutung haben:

| | |
|---|---|
| X¹ = | W, Nb, Ta, Cr und/oder Ce, |
| X² = | Cu, Ni, Co, Fe, Mn und/oder Zn, |
| X³ = | Sb und/oder Bi, |
| X⁴ = | eines oder mehrere Alkalimetalle, |
| X⁵ = | eines oder mehrere Erdalkalimetalle, |
| X⁶ = | Si, Al, Ti und/oder Zr, |
| | |
| a = | 1 bis 6, |
| b = | 0,2 bis 4, |
| c = | 0,5 bis 18, |
| d = | 0 bis 40, |
| e = | 0 bis 2, |
| f = | 0 bis 4, |
| g = | 0 bis 40 und |
| n = | eine Zahl, die durch die Wertigkeit und Häufigkeit der von Sauerstoff verschiedenen Elemente in VII bestimmt wird, |

subsumieren.

Erfindungsgemäß bevorzugte Ausführungsformen innerhalb der aktiven Multimetalloxide VII sind jene, die von den nachfolgenden Bedeutungen der Variablen der allgemeinen Formel VII erfasst werden:

| | |
|---|---|
| X¹ = | W, Nb, und/oder Cr, |
| X² = | Cu, Ni, Co, und/oder Fe, |
| X³ = | Sb, |
| X⁴ = | Na und/oder K, |
| X⁵= | Ca, Sr und/oder Ba, |
| X⁶ = | Si, Al, und/oder Ti, |
| | |
| a = | 1,5 bis 5, |
| b = | 0,5 bis 2, |
| c = | 0,5 bis 3, |
| d = | 0 bis 2, |
| e = | 0 bis 0,2, |
| f = | 0 bis 1 und |
| n = | eine Zahl, die durch die Wertigkeit und Häufigkeit der von Sauerstoff verschiedenen Elemente in VII bestimmt wird. |

Erfindungsgemäß ganz besonders bevorzugte Multimetalloxide VII sind jedoch jene der allgemeinen Formel VIII,

Mo₁₂V_{a'}Y¹_{b'}Y²_{c'}Y⁵_{f'}Y⁶_{g'}O_{n'} (VIII),

mit

| | |
|---|---|
| Y¹ = | W und/oder Nb, |
| Y² = | Cu und/oder Ni, |
| Y⁵ = | Ca und/oder Sr, |
| Y⁶ = | Si und/oder Al, |
| | |
| a' = | 2 bis 4, |
| b' = | 1 bis 1,5, |
| c' = | 1 bis 3, |
| f' = | 0 bis 0,5 |
| g' = | 0 bis 8 und |
| n' = | eine Zahl, die durch die Wertigkeit und Häufigkeit der von Sauerstoff verschiedenen Elementen in VIII bestimmt wird. |

Die erfindungsgemäß geeigneten Multimetalloxidaktivmassen (VII) sind in an sich bekannter, z. B. in der DE-A 43 35 973 oder in der EP-A 714 700 offenbarter, Weise erhältlich.

Prinzipiell können für den Schritt "Acrolein → Acrylsäure" geeignete Multimetalloxidaktivmassen, insbesondere solche der allgemeinen Formel VII, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z. B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen VII kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen VII kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die resultierenden Multimetalloxidmassen, insbesondere jene der allgemeinen Formel VII, können für die Acroleinoxidation sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z. B. durch Tablettieren, Extrudieren oder Strangpressen) Vollkatalysatoren hergestellt werden, wobei gegebenenfalls Hilfsmittel wie z. B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z. B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm (z. B. 8,2 mm oder 5,1 mm) betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z. B. aus der DE-A 2 909 671, der EP-A 293 859 oder aus der EP-A 714 700 bekannt ist.

Zweckmäßigerweise wird zur Beschichtung der Trägerkörper die aufzubringende Pulvermasse befeuchtet und nach dem Aufbringen, z. B. mittels heißer Luft, wieder getrocknet. Die Schichtdicke der auf den Trägerkörper aufgebrachten Pulvermasse wird zweckmäßigerweise im Bereich 10 bis 1000 µm, bevorzugt im Bereich 50 bis 500 µm und besonders bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z. B. Kugeln oder Hohlzylinder mit Splittauflage, bevorzugt werden. Geeignet ist die Verwendung von im wesentlichen unporösen, oberflächenrauhen, kugelförmigen Trägern aus Steatit, deren Durchmesser 1 bis 10 mm bzw. bis 8 mm, bevorzugt 4 bis 5 mm beträgt. D. h., geeignete Kugelgeometrien können Durchmesser von 8,2 mm oder von 5,1 mm aufweisen. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für den Schritt "Acrolein → Acrylsäure" zu verwendende Multimetalloxidaktivmassen sind ferner Massen der allgemeinen Formel IX,

[D]ₚ[E]_{q} (IX),

in der die Variablen folgende Bedeutung haben:

| | |
|---|---|
| D = | Mo₁₂V_{a"}Z¹_{b"}Z²_{c"}Z³_{d"}Z⁴_{e"}Z⁵_{f"}Z⁶_{g"}O_{x"}, |
| E = | Z⁷₁₂Cu_{h"}H_{i"}O_{y"}, |
| Z¹ = | W, Nb, Ta, Cr und/oder Ce, |
| Z² = | Cu, Ni, Co, Fe, Mn und/oder Zn, |
| Z³ = | Sb und/oder Bi, |
| Z⁴ = | Li, Na, K, Rb, Cs und/oder H |
| Z⁵ = | Mg, Ca, Sr und/oder Ba, |
| Z⁶ = | Si, Al, Ti und/oder Zr, |
| Z⁷ = | Mo, W, V, Nb und/oder Ta, vorzugsweise Mo und/oder W |
| | |
| a" = | 1 bis 8, |
| b" = | 0,2 bis 5, |
| c" = | 0 bis 23, |
| d" = | 0 bis 50, |
| e" = | 0 bis 2, |
| f" = | 0 bis 5, |
| g" = | 0 bis 50, |
| h" = | 4 bis 30, |
| i" = | 0 bis 20 und |
| x",y" = | Zahlen, die durch die Wertigkeit und Häufigkeit der von Sauerstoff verschiedenen Element in IX bestimmt werden und |
| p,q = | von Null verschiedene Zahlen, deren Verhältnis p/q 160:1 bis 1:1 beträgt, |

und die dadurch erhältlich sind, dass man eine Multimetalloxidmasse E

Z⁷₁₂Cu_{h"}H_{i"}O_{y"} (E),

in feinteiliger Form getrennt vorbildet (Ausgangsmasse 1) und anschließend die vorgebildete feste Ausgangsmasse 1 in eine wäßrige Lösung, eine wäßrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D

Mo₁₂V_{a"}Z¹_{b"}Z²_{c"}Z³_{d"}Z⁴_{e"}Z⁵_{f"}Z⁶_{g"} (D),

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen IX, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z. B. die EP-A 668 104, die DE-A 197 36 105, die DE-A 100 46 928, die DE-A 197 40 493 und die DE-A 195 28 646.

Hinsichtlich der Formgebung gilt bezüglich Multimetalloxidmassen IX-Katalysatoren das bei den Multimetalloxidmassen VII-Katalysatoren Gesagte.

Für den Schritt "Acrolein → Acrylsäure" in hervorragender Weise geeignete Multimetalloxidkatalysatoren sind auch jene der DE-A 198 15 281, insbesondere mit Multimetalloxidaktivmassen der allgemeinen Formel I dieser Schrift.

Vorteilhaft werden für den Schritt vom Propylen zum Acrolein Vollkatalysatorringe und für den Schritt vom Acrolein zur Acrylsäure Schalenkatalysatorringe verwendet.

Die Durchführung der Paritaloxidation des erfindungsgemäßen Verfahrens, vom Propylen zum Acrolein (sowie gegebenenfalls Acrylsäure), kann mit den beschriebenen Katalysatoren z. B. in einem Einzonen-Vielkontaktrohr-Festbettreaktor durchgeführt werden, wie ihn die DE-A 4 431 957 beschreibt. Dabei können Reaktionsgasgemisch und Wärmeträger (Wärmeaustauschmittel) über den Reaktor betrachtet im Gleichstrom oder im Gegenstrom geführt werden.

Der Reaktionsdruck liegt üblicherweise im Bereich von 1 bis 3 bar und die Gesamtraumbelastung des Katalysatorfestbetts mit Reaktionsgas(ausgangs)gemisch 2 beträgt vorzugsweise 1500 bis 4000 bzw. 6000 Nl/l•h oder mehr. Die Propylenlast (die Propylenbelastung des Katalysatorfestbetts) beträgt typisch 90 bis 200 Nl/l•h oder bis 300 Nl/l•h oder mehr. Propylenlasten oberhalb von 135 Nl/l•h bzw. ≥ 140 Nl/l•h, oder ≥ 150 Nl/l•h, oder ≥ 160 Nl/l•h sind erfindungsgemäß besonders bevorzugt, da das erfindungsgemäße Reaktionsgasausgangsgemisch infolge des Beiseins von nicht umgesetztem Propan ein günstiges Heißpunktverhalten bedingt (alles Vorgenannte gilt auch unabhängig von der speziellen Wahl des Festbettreaktors).

Bevorzugt wird der Einzonen-Vielkontaktrohr-Festbettreaktor mit dem Beschickungsgasgemisch von oben angeströmt. Als Wärmeaustauschmittel wird zweckmäßig eine Salzschmelze, bevorzugt bestehend aus 60 Gew.-% Kaliumnitrat (KNO₃) und 40 Gew.-% Natriumnitrit (NaNO₂), oder aus 53 Gew.-% Kaliumnitrat (KNO₃), 40 Gew.-% Natriumnitrit (NaNO₂) und 7 Gew.-% Natriumnitrat (NaNO₃),eingesetzt.

Über den Reaktor betrachtet können, wie bereits gesagt, Salzschmelze und Reaktionsgasgemisch sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt mit Katalysator zu beschicken (für die Anströmung von unten nach oben wird die Beschickungsabfolge in zweckmäßiger Weise umgekehrt):
- zunächst auf einer Länge von 40 bis 80 bzw. bis 60 % der Kontaktrohrlänge entweder nur Katalysator, oder ein Gemisch aus Katalysator und Inertmaterial, wobei letzteres, bezogen auf die Mischung, einen Gewichtsanteil von bis zu 30 bzw. bis zu 20 Gew.-% ausmacht (Abschnitt C);
- daran anschließend auf einer Länge von 20 bis 50 bzw. bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 40 Gew.-% ausmacht (Abschnitt B); und
- abschließend auf einer Länge von 10 bis 20 % der Gesamtrohrlänge eine Schüttung aus Inertmaterial (Abschnitt A), die vorzugsweise so gewählt wird, dass sie einen möglichst geringen Druckverlust bedingt.

### Bevorzugt ist der Abschnitt C unverdünnt.

Vorgenannte Beschickungsvariante ist insbesondere dann zweckmäßig, wenn als Katalysatoren solche gemäß Research Disclosure Nr. 497012 vom 29.08.2005, oder gemäß Beispiel 1 der DE-A 100 46 957 oder gemäß Beispiel 3 der DE-A 100 46 957 und als Inertmaterial Ringe aus Steatit mit der Geometrie 7 mm x 7 mm x 4 mm (Außendurchmesser x Höhe x Innendurchmesser) verwendet werden. Hinsichtlich der Salzbadtemperatur gilt das in der DE-A 4 431 957 Gesagte.

Die Durchführung der erfindungsgemäßen Partialoxidation, vom Propylen zum Acrolein (und gegebenenfalls Acrylsäure), kann mit den beschriebenen Katalysatoren aber auch z. B. in einem Zweizonen-Vielkontaktrohr-Festbettreaktor durchgeführt werden, wie es die DE-A 199 10 506, die DE-A 10 2005 009 885, die DE-A 10 2004 032 129, DE-A 10 2005 013 039 und die DE-A 10 2005 009 891 sowie die DE-A 10 2005 010 111 beschreibt. In beiden vorstehend beschriebenen Fällen (sowie ganz allgemein beim erfindungsgemäßen Verfahren) liegt der erzielte Propenumsatz bei einfachem Durchgang normalerweise bei Werten ≥ 90 mol-%, bzw. ≥ 95 mol-% und die Selektivität der Acroleinbildung bei Werten ≥ 90 mol-%. Erfindungsgemäß vorteilhaft erfolgt die erfindungsgemäße Partialoxidation von Propen zu Acrolein, oder Acrylsäure oder deren Gemisch wie in der EP-A 1159244 und ganz besonders bevorzugt wie in der WO 04/085363 sowie in der WO 04/085362 beschrieben.

Die Schriften EP-A 1159244, WO 04/085363 und WO 04/085362 werden als integraler Bestandteil dieser Schrift betrachtet.

D. h., die erfindungsgemäß durchzuführende Partialoxidation des Propylens lässt sich besonders vorteilhaft an einem Katalysatorfestbett mit erhöhter Propylenbelastung durchführen, das wenigstens zwei Temperaturzonen aufweist.

Diesbezüglich wird z. B. auf die EP-A 1159244 und die WO 04/085362 verwiesen.

Eine typische Zusammensetzung des Reaktionsgasausgangsgemischs für die Partialoxidation vom Propylen zur Acrolein kann beim erfindungsgemäßen Verfahren umfassen:

| | |
|---|---|
| 5 bis 9 Vol.-% | Propylen, |
| 8 bis 18 Vol.-% | molekularer Sauerstoff, |
| 6 bis 30 (bzw. bis 35) Vol.-% | Propan und |
| 32 bis 72 Vol.-% | molekularer Stickstoff. |

Die Durchführung des zweiten Schrittes im Fall einer zweistufigen Partialoxidation von Propylen zu Acrolein, nämlich die Partialoxidation vom Acrolein zur Acrylsäure, kann mit den beschriebenen Katalysatoren z. B. in einem Einzonen-Vielkontaktrohr-Festbettreaktor durchgeführt werden, wie es die DE-A 44 31 949 beschreibt. Dabei können Reaktiongsgasgemisch und Wärmeträger über den Reaktor betrachtet im Gleichstrom geführt werden. In der Regel wird das Produktgasgemisch der vorausgehenden erfindungsgemäßen Propylenpartialoxidation zu Acrolein grundsätzlich als solches (gegebenenfalls nach erfolgter Zwischenkühlung (diese kann indirekt oder direkt durch z. B. Sekundärluftzugabe erfolgen) desselben), d.h., ohne Nebenkomponentenabtrennung, in die zweite Reaktionsstufe, d.h. in die Acroleinpartialoxidation geführt.

Der für den zweiten Schritt, die Acroleinpartialoxidation, benötigte molekulare Sauerstoff kann (dabei) schon im Reaktionsgasausgangsgemisch für die erfindungsgemäße Propylenpartialoxidation zum Acrolein enthalten sein. Er kann aber auch teilweise oder vollständig dem Produktgasgemisch der ersten Reaktionsstufe, d. h., der erfindungsgemäßen Propylenpartialoxidation zum Acrolein, unmittelbar zugegeben werden (dies erfolgt vorzugsweise als (Sekundär)Luft, kann jedoch auch in Form von reinem Sauerstoff oder von Gemischen aus Inertgas oder Sauerstoff erfolgen). Unabhängig davon wie vorgegangen wird, weist das Beschickungsgasgemisch (das Reaktionsgasausgangsgemisch) einer solchen Partialoxidation des Acroleins zu Acrylsäure, mit Vorteil nachfolgende Gehalte auf:

| | |
|---|---|
| 4 bis 8 Vol.-% | Acrolein, |
| 2,25 bzw. 4,5 bis 9 Vol.-% | molekularer Sauerstoff, |
| 6 bis 30 Vol.-% | Propan, |
| 32 bis 72 Vol.-% | molekularer Stickstoff und |
| 5 bis 15 Vol.-% | Wasserdampf. |

Bevorzugt weist das vorgenannte Reaktionsgasausgangsgemisch folgende Gehalte auf:

| | |
|---|---|
| 5 bis 8 Vol.-% | Acrolein, |
| 2,75 bzw. 5,5 bis 9 Vol.-% | molekularer Sauerstoff, |
| 10 bis 25 Vol.-% | Propan, |
| 40 bis 70 Vol.-% | molekularer Stickstoff und |
| 5 bis 15 Vol.-% | Wasserdampf. |

Ganz besonders bevorzugt weist das vorgenannte Reaktiongsgasausgangsgemisch folgende Gehalte auf:

| | |
|---|---|
| 5 bis 8 Vol.-% | Acrolein (bevorzugt 6 bis 7 Vol.-%), |
| 3 bzw. 6 bis 9 Vol.-% | molekularer Sauerstoff, |
| 10 bis 20 Vol-% | Propan (bevorzugt 10 bis 16 Vol.-%), |
| 50 bis 65 Vol.-% | molekularer Stickstoff und |
| 7 bis 13 Vol.-% | Wasserdampf, |

wobei die Vorzugsbereiche unabhängig voneinander gelten, mit Vorteil jedoch simultan realisiert werden.

Wie in der ersten Reaktionsstufe (Propylen → Acrolein) liegt auch in der zweiten Reaktionsstufe (Acrolein → Acrylsäure) der Reaktionsdruck üblicherweise im Bereich von 1 bis 3 bar, und die Gesamtraumbelastung des Katalysatorfestbetts mit Reaktionsgas(ausgangs)gemisch liegt vorzugsweise bei 1500 bis 4000 bzw. 6000 Nl/l•h oder mehr. Die Acroleinlast (die Acroleinbelastung des Katalysatorfestbetts) beträgt typisch 90 bis 190 Nl/l•h, oder bis 290 Nl/l•h oder mehr. Acroleinlasten oberhalb von 135 Nl/l•h, bzw. ≥ 140 Nl/l•h, oder ≥ 150 Nl/l•h, oder ≥ 160 Nl/l•h sind besonders bevorzugt, da das erfindungsgemäß einzusetzende Reaktionsgasausgangsgemisch infolge des Beiseins von Propan ebenfalls ein günstiges Heißpunktverhalten bedingt.

Der Acroleinumsatz, bezogen auf einmaligen Durchgang des Reaktionsgasgemischs durch das Katalysatorfestbett, beträgt zweckmäßig normalerweise ≥ 90 mol-% und die damit einhergehende Selektivität der Acrylsäurebildung ≥ 90 mol-%.

Bevorzugt wird der Einzonen-Vielkontaktrohr-Festbettreaktor mit dem Beschickungsgasgemisch ebenfalls von oben angeströmt. Als Wärmeaustauschmittel wird auch in der zweiten Stufe in zweckmäßiger Weise eine Salzschmelze, bevorzugt aus 60 Gew.-% Kaliumnitrat (KNO₃) und 40 Gew.-% Natriumnitrit (NaNO₂) oder aus 53 Gew.-% Kaliumnitrat (KNO₃), 40 Gew.-% Natriumnitrit (NaNO₂) und 7 Gew.-% Natriumnitrat (Na-NO₃) bestehend, eingesetzt. Über den Reaktor betrachtet können, wie bereits gesagt, Salzschmelze und Reaktionsgasgemisch sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt zu beschicken:
- zunächst auf einer Länge von 50 bis 80 bzw. bis 70 % der Kontaktrohrlänge entweder nur Katalysator oder ein Gemisch aus Katalysator und Inertmaterial, wobei letzteres, bezogen auf die Mischung, einen Gewichtsanteil von bis zu 30 (bzw. 20) Gew.-% ausmacht (Abschnitt C);
- daran anschließend auf einer Länge von 20 bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 50 bzw. bis zu 40 Gew.-% ausmacht (Abschnitt B); und
- abschließend auf einer Länge von 5 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt. Wie ganz allgemein bei der heterogen katalysierten Gasphasenpartialoxidation von Acrolein zu Acrylsäure (insbesondere bei hohen Acroleinbelastungen des Katalysatorbetts und hohen Wasserdampfgehalten des Beschickungsgasgemischs), kann der Abschnitt B auch aus zwei aufeinanderfolgenden Katalysatorverdünnungen bestehen (zum Zweck der Minimierung von Heißpunkttempatur und Heißpunkttemperatursensitivität). Von unten nach oben zunächst mit bis zu 30 (bzw. 20) Gew.-% Inertmaterial und daran anschließend mit > 20 Gew.-% bis 50 bzw. bis 40 Gew.-% Inertmaterial. Der Abschnitt C ist dann bevorzugt unverdünnt.

Für eine Anströmung der Kontaktrohre von unten nach oben, wird die Kontaktrohrbeschickung in zweckmäßigerweise umgekehrt.

Vorgenannte Beschickungsvariante ist insbesondere dann zweckmäßig, wenn als Katalysatoren solche gemäß Herstellungsbeispiel 5 der DE-A 100 46 928 oder solche gemäß DE-A 198 15 281 und als Inertmaterial Ringe aus Steatit mit der Geometrie 7 mm x 7 mm x 4 mm oder 7 mm x 7 mm x 3 mm (jeweils Außendurchmesser x Höhe x Innendurchmessser) verwendet werden. Hinsichtlich der Salzbadtemperatur gilt das in der DE-A 443 19 49 Gesagte. Sie wird in der Regel so gewählt, daß der erzielte Acroleinumsatz bei einfachem Durchgang normalerweise ≥ 90 mol-%, bzw. ≥ 95 mol-% oder ≥ 99 mol-% beträgt.

Die Durchführung der Partialoxidation vom Acrolein zur Acrylsäure, kann mit den beschriebenen Katalysatoren aber auch z. B. in einem Zweizonen-Vielkontaktrohr-Festbettreaktor durchgeführt werden, wie es die DE-A 199 10 508 beschreibt. Für den Acroleinumsatz gilt das oben Genannte. Auch im Fall der Durchführung einer wie vorstehend beschriebenen Acroleinpartialoxidation als zweite Reaktionsstufe einer zweistufigen Propylenoxidation zu Acrylsäure in einem Zweizonen-Vielkontaktrohr-Festbettreaktor wird man zur Erzeugung des Beschickungsgasgemisches (Reaktionsgasausgangsgemischs) zweckmäßig unmittelbar das Produktgasgemisch der auf den ersten Schritt gerichteten Partialoxidation (gegebenenfalls nach erfolgter indirekter oder direkter (z. B. durch Zufuhr von Sekundärluft) Zwischenkühlung desselben) verwenden (wie sie vorstehend bereits beschrieben wurde). Der für die Acroleinpartialoxidation benötigte Sauerstoff wird vorzugsweise als Luft (gegebenenfalls aber auch als reiner molekularer Sauerstoff oder als Gemisch aus molekularem Sauerstoff und einem Inertgas) zugesetzt und z. B. dem Produktgasgemisch des ersten Schritts der zweistufigen Partialoxidation (Propylen → Acrolein) unmittelbar zugegeben. Er kann aber auch, wie bereits beschrieben, bereits im Reaktionsgasausgangsgemisch für die erste Reaktionsstufe enthalten sein.

Bei einer zweistufigen Partialoxidation von Propylen zu Acrylsäure mit unmittelbarer Weiterverwendung des Produktgasgemisches des ersten Schritts der Partialoxidation zur Beschickung des zweiten Schritts der Partialoxidation, wird man in der Regel zwei Einzonen-Vielkontaktrohr-Festbettreaktoren (bei hoher Reaktandenbelastung des Katalysatorbetts ist, wie ganz allgemein gültig, eine Gleichstromfahrweise zwischen Reaktionsgas und Salzbad (Wärmeträger) über den Rohrbündelreaktor betrachtet bevorzugt) oder zwei Zweizonen-Vielkontaktrohr-Festbettreaktoren hintereinanderschalten. Eine gemischte Hintereinanderschaltung (Einzonen/Zweizonen oder umgekehrt) ist auch möglich.

Zwischen den Reaktoren kann sich ein Zwischenkühler befinden, der gegebenenfalls Inertschüttungen enthalten kann, die eine Filterfunktion ausüben können. Die Salzbadtemperatur von Vielkontaktrohrreaktoren für den ersten Schritt der zweistufigen Partialoxidation von Propylen zu Acrylsäure beträgt in der Regel 300 bis 400°C. Die Salzbadtemperatur von Vielkontaktrohrreaktoren für den zweiten Schritt der Partialoxidation von Propylen zu Acrylsäure, die Partialoxidation von Acrolein zu Acrylsäure, beträgt meist 200 bis 350°C. Außerdem werden die Wärmeaustauschmittel (bevorzugt Salzschmelzen) normalerweise in solchen Mengen durch die relevanten Vielkontaktrohr-Festbettreaktoren geführt, dass der Unterschied zwischen ihrer Eingangs- und ihrer Ausgangstemperatur in der Regel ≤ 5°C beträgt. Wie bereits erwähnt, können beide Schritte der Partialoxidation von Propylen zu Acrylsäure aber auch wie in der DE-A 101 21 592 beschrieben in einem Reaktor an einer Beschickung vollzogen werden.

Es sei auch nochmals erwähnt, dass ein Teil des Reaktionsgasausgangsgemischs für den ersten Schritt ("Propylen → Acrolein") aus der Partialoxidation kommendes Restgas sein kann.

Hierbei handelt es sich, wie bereits gesagt, um ein molekularen Sauerstoff enthaltendes Gas, das nach der Zielproduktabtrennung (Acrolein- und/oder Acrylsäureabtrennung) vom Produktgasgemisch der Partialoxidation verbleibt und teilweise als inertes Verdünnungsgas in die Beschickung für den ersten und/oder gegebenenfalls zweiten Schritt der Partialoxidation von Propylen zu Acrolein und/oder Acrylsäure rückgeführt werden kann.

Bevorzugt wird solches Propan, molekularen Sauerstoff und gegebenenfalls nicht umgesetztes Propylen enthaltendes Restgas erfindungsgemäß jedoch vorteilhaft ausschließlich als gasförmiger Ausgangsstrom 2 in die heterogen katalysierte Propandehydrierung in der Reaktionszone A rückgeführt.

Insgesamt bildet ein Rohrbündelreaktor innerhalb dessen sich die Katalysatorbeschickung längs der einzelnen Kontaktrohre mit Beendigung des ersten Reaktionsschrittes entsprechend ändert (derartige zweistufige Propylenpartialoxidationen im sogenannten "Single-Reactor" lehren z. B. die EP-A 91 13 13, die EP-A 97 98 13, die EP-A 99 06 36 und die DE-A 28 30 765) die einfachste Realisierungsform zweier Oxidationsstufen für die beiden Schritte der Partialoxidation von Propylen zu Acrylsäure. Gegebenenfalls wird dabei die Beschickung der Kontaktrohre mit Katalysator durch eine Inertmaterialschüttung unterbrochen.

Vorzugsweise werden die beiden Oxidationsstufen jedoch in Form zweier hintereinander geschalteter Rohrbündelsysteme realisiert. Diese können sich in einem Reaktor befinden, wobei der Übergang von einem Rohrbündel zum anderen Rohrbündel von einer nicht im Kontaktrohr untergebrachten (zweckmäßigerweise begehbaren) Schüttung aus Inertmaterial gebildet wird. Während die Kontaktrohre in der Regel von einem Wärmeträger umspült werden, erreicht dieser eine wie vorstehend beschrieben angebrachte Inertmaterialschüttung nicht. Mit Vorteil werden daher die beiden Kontaktrohrbündel in räumlich voneinander getrennten Reaktoren untergebracht. In der Regel befindet sich dabei zwischen den beiden Rohrbündelreaktoren ein Zwischenkühler, um eine gegebenenfalls erfolgende Acroleinnachverbrennung im Produktgasgemisch, das die erste Oxidationszone verlässt, zu mindern. Die Reaktionstemperatur in der ersten Reaktionsstufe (Propylen → Acrolein) liegt in der Regel bei 300 bis 450°C, bevorzugt bei 320 bis 390°C. Die Reaktionstemperatur in der zweiten Reaktionsstufe (Acrolein → Acrylsäure) liegt in der Regel bei 200 bis 370°C, häufig bei 220 bis 330°C. Der Reaktionsdruck beträgt in beiden Oxidationszonen zweckmäßig 0,5 bis 5, vorteilhaft 1 bis 3 bar. Die Belastung (Nl/l•h) der Oxidatiohskatalysatoren mit Reaktionsgas beträgt in beiden Reaktionsstufen häufig 1500 bis 2500 Nl/l•h bzw. bis 4000 Nl/l•h. Die Belastung mit Propylen kann dabei bei 100 bis 200 oder 300 und mehr Nl/l•h liegen.

Prinzipiell können die beiden Oxidationsstufen beim erfindungsgemäßen Verfahren so gestaltet werden, wie es z. B. in der DE-A 198 37 517, der DE-A 199 10 506, der DE-A 199 10 508 sowie der DE-A 198 37 519 beschrieben ist.

In beiden Reaktionsstufen wirkt sich dabei ein Überschuss an molekularem Sauerstoff relativ zur reaktionsstöchiometrisch erforderlichen Menge vorteilhaft auf die Kinetik der jeweiligen Gasphasenpartialoxidation sowie auf die Katalysatorstandzeit aus.

Prinzipiell ist die erfindungsgemäß durchzuführende heterogen katalysierte Gasphasen-Partialoxidation von Propylen zu Acrylsäure aber auch in einem einzigen Einzonenrohrbündelreaktor wie folgt realisierbar. Beide Reaktionsschritte erfolgen in einem Oxidationsreaktor der mit einem oder mehreren Katalysatoren beschickt ist, deren Aktivmasse ein die Elemente Mo, Fe und Bi enthaltendes Multimetalloxid ist, das die Umsetzung beider Reaktionsschritte zu katalysieren vermag. Selbstredend kann sich auch diese Katalysatorbeschickung längs der Reaktionskoordinate kontinuierlich oder abrupt ändern. Natürlich kann bei einer Ausführungsform einer erfindungsgemäßen zweistufigen Partialoxidation von Propylen zu Acrylsäure in Gestalt zweier hintereinandergeschalteter Oxidationsstufen aus dem das die erste Oxidationsstufe verlassenden Produktgasgemisch in selbigem enthaltenes, in der ersten Oxidationsstufe als Nebenprodukt entstandenes, Kohlenoxid und Wasserdampf bei Bedarf vor der Weiterleitung in die zweite Oxidationsstufe teilweise oder vollständig abgetrennt werden. Vorzugsweise wird man erfindungsgemäß eine Verfahrensweise wählen, die solch einer Abtrennung nicht vorsieht.

Als Quellen für eine zwischen beiden Oxidationsstufen durchgeführte Sauerstoffzwischeneinspeisung kommen, wie bereits gesagt, neben Luft (bevorzugt) sowohl reiner molekularer Sauerstoff als auch mit Inertgas wie CO₂, CO, Edelgasen, N₂ und/oder gesättigten Kohlenwasserstoffen verdünnter molekularer Sauerstoff in Betracht.

Durch Zudosieren von z. B. kalter Luft zum Produktgasgemisch der ersten Partialoxidaitonsstufe kann im Rahmen des erfindungsgemäßen Verfahrens auch auf direktem Weg eine Abkühlung desselben bewirkt werden, bevor es als Bestandteil eines Reaktionsgasausgangsgemisch für die zweite Partialoxidationsstufe weiterverwendet wird.

Erfindungsgemäß vorteilhaft erfolgt die Partialoxidation von Acrolein zu Acrylsäure wie in der EP-A 11 59 246, und ganz besonders bevorzugt wie in der WO 04/085365 sowie in der WO 04/085370 beschrieben. Erfindungsgemäß bevorzugt wird dabei als Acrolein enthaltendes Reaktionsgasausgangsgemisch jedoch ein Reaktionsgasausgangsgemisch verwendet, das das Produktgasgemisch einer erfindungsgemäßen Erststufenpartialoxidation von Propylen zu Acrolein ist, das gegebenenfalls mit soviel Sekundärluft ergänzt wurde, dass das Verhältnis von molekularem Sauerstoff zu Acrolein in dem resultierenden Reaktionsgasausgangsgemisch in jedem Fall 0,5 bis 1,5 beträgt. Die Schriften EP-A 1159246, WO 04/08536 und WO 04/085370 werden als integraler Bestandteil dieser Schrift betrachtet.

D.h., die erfindungsgemäße Partialoxidation von Acrolein zu Acrylsäure lässt sich vorteilhaft an einem Katalysatorfestbett mit erhöhter Acroleinbelastung durchführen, das wenigstens zwei Temperaturzonen aufweist.

Insgesamt wird man eine zweistufige Partialoxidation von Propylen zu Acrylsäure bevorzugt wie in der EP-A 1 159 248 bzw. in der WO 04/085367 oder WO 04/085369 beschrieben durchführen.

Der die erfindungsgemäß durchzuführende Partialoxidation (nach der ersten und/oder zweiten Reaktionsstufe) verlassende Produktgasgemischstrom B ist im Fall einer Herstellung von Acrolein und/oder Acrylsäure in der Regel im wesentlichen zusammengesetzt aus dem Zielprodukt Acrolein oder Acrylsäure oder dessen Gemisch mit Acrolein, nicht umgesetztem molekularem Sauerstoff (mit Blick auf die Lebensdauer der verwendeten Katalysatoren ist es günstig, wenn der Sauerstoffgehalt im Produktgasgemisch beider Partialoxidationsstufen noch wenigstens 1,5 bis 4 Vol.-% beträgt), Propan, nicht umgesetztem Propylen, molekularem Stickstoff, als Nebenprodukt entstandenem und/oder als Verdünnungsgas mitverwendetem Wasserdampf, als Nebenprodukt entstandenen und/oder als Verdünnungsgas mitverwendeten Kohlenoxiden, sowie geringen Mengen sonstiger niederer Aldehyde, niederer Alkancarbonsäuren (z. B. Essigsäure, Ameisensäure und Propionsäure) sowie Maleinsäureanhydrid, Benzaldehyd, aromatische Carbonsäuren und aromatische Carbonsäureanhydride (z.B. Phthalsäureanhydrid und Benzoesäure), gegebenenfalls weiteren Kohlenwasserstoffen, wie z. B. C4-Kohlenwasserstoffe (z. B. Buten-1 und eventuell sonstige Butene), und anderen inerten Verdünnungsgasen.

Das Zielprodukt kann aus dem Produktgasgemisch B in an sich bekannter Weise in einer zweiten Trennzone B abgetrennt werden (z. B. durch partielle oder vollständige sowie gegebenenfalls fraktionierende Kondensation der Acrylsäure oder durch Absorption von Acrylsäure in Wasser oder in einem hochsiedenden hydrophoben organischen Lösungsmittel oder durch Absorption von Acrolein in Wasser oder in wässrigen Lösungen niederer Carbonsäuren sowie anschließende Aufarbeitung der Kondensate und/oder Absorbate; erfindungsgemäß bevorzugt wird das Produktgasgemisch B fraktionierend kondensiert werden; vgl. z. B. EP-A 1 388 533, EP-A 1 388 532, DE-A 102 35 847, EP-A 792 867, WO 98/01415, EP-A 1 015 411, EP-A 1 015 410, WO 99/50219, WO 00/53560, WO 02/09839, DE-A 102 35 847, WO 03/041833, DE-A 102 23 058, DE-A 102 43 625, DE-A 103 36 386, EP-A 854 129, US-A 4,317,926, DE-A 198 37 520, DE-A 196 06 877, DE-A 190 50 1325, DE-A 102 47 240, DE-A 197 40 253, EP-A 695 736, EP-A 982 287, EP-A 1 041 062, EP-A 1 171 46, DE-A 43 08 087, DE-A 43 35 172, DE-A 44 36 243, DE-A 199 24 532, DE-A 103 32 758 sowie DE-A 199 24 533). Eine Acrylsäureabtrennung kann auch wie in der EP-A 982 287, der EP-A 982 289, der DE-A 103 36 386, der DE-A 101 15 277, der DE-A 196 06 877, der DE-A 197 40 252, der DE-A 196 27 847, der EP-A 920 408, der EP-A 1 068 174, der EP-A 1 066 239, der EP-A 1 066 240, der WO 00/53560, der WO 00/53561, der DE-A 100 53 086 und der EP-A 982 288 vorgenommen werden. Vorzugsweise wird wie in Fig. 7 der WO/0196271 bzw. wie in der DE-A 10 2004 032 129 und deren äquivalenten Schutzrechten beschrieben abgetrennt. Günstige Abtrennweisen sind auch die in den Schriften WO 04/063138, WO 04/35514, DE-A 102 43 625 und DE-A 102 35 847 beschriebenen Verfahren. Die Weiterverarbeitung einer dabei gewonnenen rohen Acrylsäure kann z. B. wie in den Schriften WO 01/77056, WO 03/041832, WO 02/055469, WO 03/078378 und WO 03/041833 beschrieben erfolgen.

Gemeinsames Merkmal der vorgenannten Trennverfahren ist (wie eingangs bereits erwähnt), dass am Kopf der jeweiligen trennwirksame Einbauten enthaltenden Trennkolonne, in deren unteren Teil das Produktgasgemisch B, normalerweise nach vorheriger direkter und/oder indirekter Kühlung desselben, zugeführt wird, normalerweise ein Restgasstrom verbleibt, der im wesentlichen diejenigen Bestandteile des Produktgasgemischs B enthält, deren Siedepunkt bei Normaldruck (1 bar) ≤ -30°C beträgt (d. h., die schwer kondensierbaren oder auch leicht flüchtigen Bestandteile).

Im unteren Teil der Trennkolonne fallen normalerweise die schwerer flüchtigen Bestandteile des Produktgasgemischs B, einschließlich des jeweiligen Zielprodukts, in kondensierter Phase an.

Die Restgasbestandteile sind in erster Linie Propan, gegebenenfalls in der Partialoxidation nicht umgesetztes Propylen, molekularer Sauerstoff sowie häufig die in der Partialoxidation sonstigen mitverwendeten inerten Verdünnungsgase, wie z. B. Stickstoff und Kohlendioxid. Wasserdampf kann je nach angewandtem Trennverfahren im Restgas nur noch in Spuren oder in Mengen von bis zu 20 Vol.-% oder mehr enthalten sein.

Von diesem Hauptrestgas wird erfindungsgemäß üblicherweise wenigstens eine (vorzugsweise Restgaszusammensetzung aufweisende) Propan, molekularen Sauerstoff und gegebenenfalls nicht umgesetztes Propylen enthaltende Teilmenge (vorzugsweise die Gesamtmenge, gegebenenfalls aber auch nur die Hälfte, oder zwei Drittel, oder drei Viertel dieser Gesamtmenge) als gasförmiger Ausgangsstrom 2 in die Reaktionszone A rückgeführt. Restgasteilmengen können aber auch in eine oder in beide Stufen der Partialoxidation rückgeführt und/oder zum Zweck der Energieerzeugung verbrannt werden.

Natürlich können auch, wie in dieser Schrift sowie in der EP-A 117 146, US-A 3,161,670, DE-A 33 13 573 und DE-A 103 16 039 beschrieben, vorab einer Verwendung von Restgas als gasförmigen Ausgangsstrom 2 aus dem Restgas insbesondere von Propan, Propylen und molekularem Sauerstoff verschiedene Bestandteile teilweise oder vollständig abgetrennt werden.

Bei der Aufarbeitung der kondensierten Phase (zum Zweck der Abtrennung des Zielproduktes) können weitere restliche Gase anfallen, da normalerweise versucht werden wird, die insgesamt im Produktgasgemisch B enthaltene Menge an nicht umgesetztem Propan in die Reaktionszone A zurückzuführen und im Rahmen der Zielproduktabtrennung wiederzugewinnen. Diese enthalten in der Regel zwar noch Propan sowie gegebenenfalls Propylen, häufig jedoch keinen molekularen Sauerstoff mehr. Üblicherweise werden sie mit dem Hauptrestgas zu einem Gesamtrestgas vereint als gasförmiger Ausgangsstrom 2 in die Reaktionszone A rückgeführt. Es ist aber auch eine separate Verwertung bzw. Rückführung in die Reaktionszone A solcher weiteren restliche Gase möglich.

Durch die vorzugsweise vollständige Rückführung des Gesamtrestgases kann so im kontinuierlichen Betrieb eine kontinuierliche Umsetzung von Propan zu Acrylsäure und/oder Acrolein erfolgen.

Wesentlich ist dabei, dass durch die beschriebene Rückführung sowie durch die erfindungsgemäße Betriebsweise der Reaktionszone A in letzterer eine Überführung von Frischpropan zu Propylen mit nahezu hundertprozentiger Selektivität erreichbar ist.

Die Vorteilhaftigkeit einer solchen Verfahrensweise ist dabei sowohl bei niederen (≤ 30 mol-%) als auch bei hohen (≥ 30 mol-%) Dehydrierumsätzen (bezogen auf einmaligen Durchgang durch die Reaktionszone A) gegeben. Generell ist es erfindungsgemäß günstig, wenn der Wasserstoffgehalt im Reaktionsgasgemischeingangsstrom A in einem wenigstens stöchiometrischen Verhältnis (bezüglich einer Sauerstoffverbrennung zu Wasser) zur in ihm enthaltenen Sauerstoffmenge steht.

An dieser Stelle sei nochmals festgehalten, dass die Abtrennung von Acrylsäure aus einem erfindungsgemäß erhaltenen Produktgasgemisch B bevorzugt so erfolgt, dass man das zuvor gegebenenfalls durch direkte und/oder indirekte Kühlung abgekühlte Produktgasgemisch B in einer trennwirksame Einbauten enthaltenden Kolonne unter Seitenabzug einer rohen Acrylsäure (z. B. in sich selbst) aufsteigend fraktionierend kondensiert und/oder mit Wasser bzw. wässriger Lösung absorbiert, wie es die WO 2004/035514 und die DE-A 102 43 625 beispielhaft beschreiben. Die entnommene rohe Acrylsäure wird nachfolgend bevorzugt einer Suspensionskristallisation unterworfen und das dabei gebildete Acrylsäuresuspensionskristallisat bevorzugt mittels einer Waschkolonne von verbliebener Mutterlauge abgetrennt. Mit Vorteil wird dabei als Waschflüssigkeit die Schmelze von in der Waschkolonne vorab abgetrennten Acrylsäurekristallen verwendet. Ferner ist die Waschkolonne bevorzugt eine solche mit erzwungenem Transport des Kristallbetts. Besonders bevorzugt handelt es sich um eine hydraulische oder um eine mechanische Waschkolonne. Im einzelnen kann der Beschreibung der WO 01/77056, der WO 03/041832 sowie der WO 03/041833 gefolgt werden. D. h., vorzugsweise wird verbliebene Mutterlauge in die fraktionierende Kondensation rückgeführt (vgl. auch EP-A 10 15 410). Der Nebenkomponentenauslaß ist normalerweise unterhalb des Seitenabzugs der rohen Acrylsäure als purge-Strom.

Unter Anwendung von lediglich einer Kristallisationsstufe ist so Acrylsäure mit einer Reinheit ≥ 99,8 Gew.-% erhältlich, die sich in hervorragender Weise zur Herstellung von Superabsorbern auf Basis von Poly-Na-Acrylat eignet.

Beispiel und Vergleichsbeispiel (konstruktives Material: Edelstahl vom Typ 1.4841)

### I. Allgemeiner Versuchsaufbau der Reaktionszone A und deren Betriebsweise

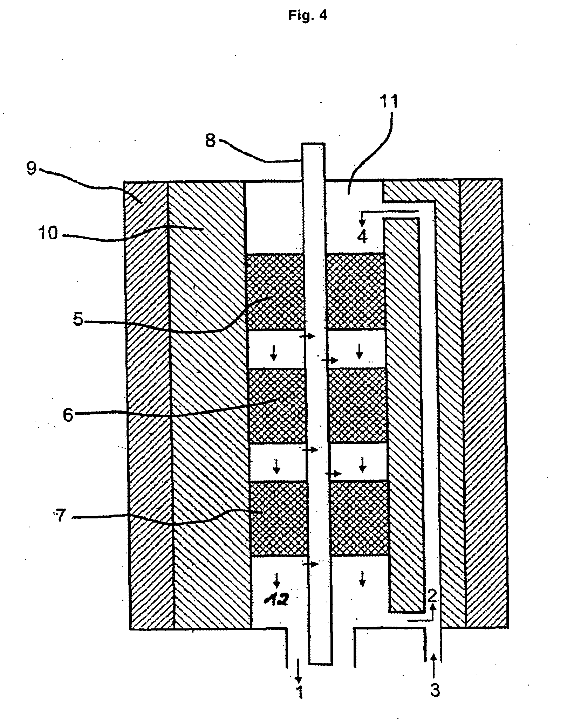

Die heterogen katalysierte partielle Propandehydrierung wird in einem Hordenschlaufenreaktor gemäß Figur 4 durchgeführt, auf die sich die numerischen Adressen im folgenden beziehen.

Ein vertikal stehender Rohrreaktor (11) (Innendurchmesser: 80 mm) ist mit einer thermischen Isolierung (10) versehen in eine Stützheizung (9) eingehaust (ermöglicht weitgehende Adiabasie des Rohrreaktors). Die Temperatur der Stützheizung beträgt 500°C. Mittig im Rohrreaktor befindet sich ein Zentralrohr (Außendurchmesser: 20 mm), welches eine Hülse für ein Zugthermoelement und eine Hülse für ein Stufenthermoelement enthält. Zusätzlich enthält es in den Rohrreaktor führende Leitungen, über die aus dem Rohrreaktor Reaktionsgasproben entnommen werden können, sowie in den Rohrreaktor führende Leitungen, über die in den Rohrreaktor Luft eingedüst werden kann.

Der Rohrreaktor enthält drei Horden (5, 6, 7), die aus drei identischen, auf einem Edelstahldrahtnetz aufgebrachten Schüttungen aus (in Strömungsrichtung in der genannten Abfolge angeordnet) Inertmaterial (Schütthöhe: 100 mm; Steatitkugeln des Durchmessers 1,5 bis 2,5 mm) und einer Mischung (Schütthöhe: 165 mm) aus Dehydrierkatalysator und Steatitkugeln (1,5 bis 2,5 mm Durchmesser) im Schüttvolumenverhältnis 1:1 bestehen. Die Gesamtschütthöhe beträgt damit jeweils 265 mm.

Der Dehydrierkatalysator ist eine Pt/Sn-Legierung, die mit den Elementen Cs, K und La in oxidischer Form promoviert ist und die auf die äußere und innere Oberfläche von ZrO₂ • SiO₂ Mischoxidträgerstränglingen (mittlere Länge (gaußverteilt im Bereich von 3 mm bis 12 mm mit Maximum bei ca. 6 mm) : 6 mm, Durchmesser: 2 mm) in der Elementstöchiometrie (Massenverhältnisse einschließlich Träger) Pt_{0,3}Sn_{0,6}La_{3,0}Cs_{0,5}K_{0,2}(ZrO₂)_{88,3}(SiO₂)_{7,1} aufgebracht ist (Katalysatorvorläuferherstellung und Aktivierung zum aktiven Katalysator wie in Beispiel 4 der DE-A 10219879).

Vor jeder Katalyastorhorde ist ein Mischelement angebracht. Das aus der letzten Horde austretende Produktgasgemisch A (12) wird in zwei Hälften identischer Zusammensetzung aufgeteilt. Die eine Hälfte (2) wird (der Produktgasgemisch A-Teilstrom 1) als Bestandteil des Reaktionsgasgemischeingangsstroms A (4) in die Dehydrierung rückgeführt. Die andere Hälfte (1) wird (der Produktgasgemisch A-Teilstrom 2) aus der Dehydrierzone (der Reaktionszone A) herausgeführt.

Der Reaktionsgasgemischeingangsstrom A (4) besteht aus dem gasförmigen Ausgangsstrom 1 (2) sowie aus dem gasförmigen Ausgangsgemischstrom (3), der aus Wasserdampf, Restgas aus der Partialoxidation, Frischpropan und molekularem Wasserstoff zusammengesetzt ist. Dieser Ausgangsgemischstrom (3) ist Treibstrahl einer Strahlpumpe, die den Produktgasgemischstrom A (12) wie beschrieben teilt und den Reaktionsgasgemischeingangsstrom A (4) erzeugt.

Die Belastung der Katalysatorgesamtmenge (ohne Inertmaterial gerechnet) aller Horden mit Propan beträgt stets 350 Nl/l•h.

Der Eingangsdruck des Reaktionsgasgemischeingangsstroms A liegt bei 2,3 bar. Seine Temperatur beträgt 500°C. Der Druckverlust über den Dehydrierreaktor beträgt ca. 200 mbar. Vor (jeweils vor dem Mischelement) der zweiten und vor der dritten Katalysatorschüttung (in Strömungsrichtung) wird dem Reaktionsgasgemisch Luft (500°C, Reaktionsdruck) zugedüst. Die Menge ist so bemessen, dass die höchste Temperatur in der jeweils nachfolgenden Katalysatorschüttung 575 bis 580°C beträgt.

### II. Allgemeiner Versuchsaufbau der heterogen katalysierten zweistufigen Partialoxidation von Propylen zu Acrylsäure und deren Betriebsweise

### Versuchsanordnung

### Erste Reaktionsstufe:

Ein Reaktionsrohr (V2A Stahl; 30 mm Außendurchmesser, 2 mm Wandstärke, 28 mm Innendurchmesser, Länge: 350 cm, sowie ein in der Reaktionsrohrmitte zentriertes Thermorohr (10 mm Außendurchmesser) zur Aufnahme eines Thermoelements mit dem die Temperatur im Reaktionsrohr auf seiner gesamten Länge ermittelt werden kann) wird von oben nach unten wie folgt beschickt:

| | |
|---|---|
| Abschnitt 1: | 50 cm Länge Steatitringe der Geometrie 7 mm x 7 mm x 4 mm (Außendurchmesser x Länge x Innendurchmesser) als Vorschüttung. |
| Abschnitt 2: | 140 cm Länge Katalysatorbeschickung mit einem homogenen Gemisch aus 20 Gew.-% (alternativ 30 Gew.-%) an Steatitringen der Geometrie 5 mm x 3 mm x 2 mm (Außendurchmesser x Länge x Innendurchmesser) und 80 Gew.-% (alternativ 70 Gew.-%) Vollkatalysator aus Abschnitt 3. |
| Abschnitt 3: | 160 cm Länge Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: [Bi₂W₂O₉ x 2WO₃]_{0,5} [Mo₁₂Co_{5,5}Fe_{2,94}Si_{1,59}K_{0,08}Oₓ]₁). Alternativ kann hier auch einer der Katalysatoren BVK1 bis BVK11 aus Research Disclosure Nr. 4497012 vom 29.08.2005 eingesetzt werden. |

Von oben nach unten werden die ersten 175 cm mittels eines im Gegenstrom gepumpten Salzbades A thermostatisiert. Die zweiten 175 cm werden mittels eines im Gegenstrom gepumpten Salzbades B thermostatisiert.

### Zweite Reaktionsstufe:

Ein Reaktionsrohr (V2A Stahl; 30 mm Außendurchmesser, 2 mm Wandstärke, 28 mm Innendurchmesser, Länge: 350 cm, sowie ein in der Reaktionsrohrmitte zentriertes Thermorohr (10 mm Außendurchmesser) zur Aufnahme eines Thermoelements mit dem die Temperatur im Reaktionsrohr auf seiner gesamten Länge ermittelt werden kann) wird von oben nach unten wie folgt beschickt:

| | |
|---|---|
| Abschnitt 1: | 20 cm Länge |
| | Steatitringe der Geometrie 7 mm x 7 mm x 4 mm (Außendurchmesser x Länge x Innendurchmesser) als Vorschüttung. |
| Abschnitt 2: | 90 cm Länge Katalysatorbeschickung mit einem homogenen Gemisch aus 25 Gew.-% (alternativ 30 Gew.-%) Steatit-Ringen der Geometrie 7 mm x 3 mm x 4 mm (Außendurchmesser x Länge x Innendurchmesser) und 75 Gew.-% (alternativ 70 Gew.-%) Schalenkatalysator aus Abschitt 4. |
| Abschnitt 3: | 50 cm Länge Katalysatorbeschickung mit einem homogenen Gemisch aus 15 Gew.-% (alternativ 20 Gew.-%) an Steatit-Ringen der Geometrie 7 mm x 3 mm x 4 mm (Außendurchmesser x Länge x Innendurchmesser) und 85 Gew.-% (alternativ 80 Gew.-%) Schalenkatalysator aus Abschnitt 4. |
| Abschnitt 4: | 190 cm Länge Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: Mo₁₂V₃W_{1,2}Cu_{2,4}Oₓ). |

Von oben nach unten werden die ersten 175 cm mittels eines im Gegenstrom gepumpten Salzbades C thermostatisiert. Die zweiten 175 cm werden mittels eines im Gegenstrom gepumpten Salzbades D thermostatisiert.

### III. Verfahren zur Herstellung von Acrylsäure aus Propan (beschrieben wird der stationäre Betriebszustand) in einer neuen Versuchsanlage

Dem ersten Katalysatorbett eines wie in I beschriebenen Hordenreaktor wird ein Reaktionsgasgemischeingangsstrom A zugeführt, der folgende Gehalte aufweist (Vol.-% bezogen auf Gesamtgas):

| | Vol.-% |
|---|---|
| Acrylsäure | 0,01 |
| Essigsäure | 0,015 |
| Wasser | 9,23 |
| 1-Buten | 0,01 |
| iso-Buten | 0,02 |
| Propan | 18,46 |
| Propylen | 3,98 |
| Ethan | 1,16 |
| Ethylen | 0,22 |
| CO₂ | 2,34 |
| CO | 0,26 |
| N₂ | 59,7 |
| O₂ | 1,62 |
| CH₄ | 0,12 |
| H₂ | 2,83 |

Er wird erzeugt (er enthält) aus (in der Reihenfolge Restgas (23°C, 3,1 bar), Frischpropan, (25°C, 4 bar), Wasserstoff (25°C, 8 bar), Wasserdampf (200°C, 2,5 bar), Dehydrierkreisgas (600°C, 1,9 bar)):
- 41,9 Vol.-% Restgas aus der Partialoxidation (gasförmigem Ausgangsstrom 2) das folgende Gehalte aufweist:

| | Vol.-% |
|---|---|
| Acrylsäure | 0,02 |
| Essigsäure | 0,04 |
| H₂O | 2,73 |
| iso-Buten | 0,01 |
| Acrolein | 0,05 |
| Propan | 17,30 |
| Propylen | 0,32 |
| Ethan | 1,20 |
| Ethylen | 0,22 |
| CO₂ | 2,41 |
| CO | 0,61 |
| N₂ | 71,21 |
| O₂ | 3,87 |

- 3,9 Vol.-% Frischpropan (gasförmiger Ausgangsstrom 3), das folgende Gehalte aufweist:

| | Vol.-% |
|---|---|
| Propan | 98,91 |
| iso-Butan | 0,05 |
| Propylen | 0,1 |
| Ethan | 0,92 |
| Ethylen | 0,01 |

- 1,02 Vol.-% molekularem Wasserstoff (gasförmiger Ausgangsstrom 4)
- 2,03 Vol.-% Wasserdampf (gasförmiger Ausgangsstrom 5) und
- 51,15 Vol.-% Dehydrierkreisgas (Produktgasgemisch A-Teilstrom 1 bzw. gasförmiger Ausgangsstrom 1)

Restgas, Frischpropan, Wasserstoff und Wasserdampf werden dabei in der genannten Reihenfolge zum Treibstrahlgemischstrom vereinigt und durch indirekten Wärmeaustausch mit dem Produktgasgemisch A-Teilstrom 2 auf 500°C, 2,3 bar gebracht.

Der resultierende Produktgasgemisch A-Teilstrom 2 weist folgende Gehalte auf :

| | Vol.-% |
|---|---|
| H₂O | 11,84 |
| iso-Buten | 0,01 |
| Propan | 14,32 |
| Propylen | 7,52 |
| Ethan | 1,21 |
| Ethylen | 0,26 |
| CO₂ | 2,61 |
| N₂ | 58,41 |
| O₂ | 0,23 |
| H₂ | 3,55 |

Der im Produktgasgemisch A-Teilstrom 2 enthaltene Propan und Propylen wird durch Absorption in technischem Tetradecan der Fa. Haltermann, DE vom Typ PKWF 4/7 af als Absorptionsmittel absorptiv abgetrennt und mittels Luft unter Erhalt des folgenden Beschickungsgases für die Partialoxidation wieder freigestrippt (dabei wird wie in der DE-A 10 2004 032 129 beschrieben verfahren), das die folgenden Gehalte aufweist:

| | Vol.-% |
|---|---|
| H₂O | 2,39 |
| Tetradekan | 0,01 |
| iso-Buten | 0,01 |
| Propan | 15,15 |
| Propylen | 7,95 |
| Ethan | 1,10 |
| Ethylen | 0,20 |
| CO₂ | 1,05 |
| N₂ | 56,99 |
| O₂ | 15,16 |

Mit diesem Beschickungsgasgemisch (es liegt außerhalb des Explosionsbereichs) wird die beschriebene erste Partialoxidationsreaktionsstufe beschickt. Die Propylenbelastung der Festbettkatalysatorbeschickung wird zu 185 Nl/l • h gewählt. Der Druck am Eingang der ersten Reaktionsstufe beträgt 3,1 bar. T_{A} = 322°C; T_{B} = 328°C.

Das die erste Reaktionsstufe verlassende Produktgasgemisch weist folgende Gehalte auf:

| | Vol.-% |
|---|---|
| Acrylsäure | 0,46 |
| Essigsäure | 0,14 |
| H₂O | 10,65 |
| 1-Buten | 0,01 |
| Acrolein | 6,99 |
| Propan | 15,16 |
| Propylen | 0,17 |
| Ethan | 1,10 |
| Ethylen | 0,20 |
| CO₂ | 1,62 |
| CO | 0,23 |
| N₂ | 57,02 |
| O₂ | 6,25 |

U^{P}_{A}, der Propenumsatz am Ende der Reaktionszone A, beträgt 64,5 mol.-%.
U^{P}_{B}, der Propenumsatz am Ende der Reaktionszone B, beträgt 94,9 mol.-%.

Dem Produktgasgemisch der ersten Stufe wird soviel Luft (25°C) zudosiert, dass das molare Verhältnis O₂ : Acrolein im resultierenden Gemisch 1,25 beträgt.

Mit diesem Gemisch wird dann die zweite Reaktionsstufe unmittelbar beschickt (T = 231,7°C).
Die Acroleinbelastung des Katalysatorfestbetts beträgt 152 Nl/l • h.
T_{C} = 263°C; T_{D} = 269°C. Der Druck am Eingang der zweiten Reaktionsstufe beträgt 2,1 bar.

Das die zweite Reaktionsstufe verlassende Produktgasgemisch weist folgende Gehalte auf:

| | Vol.-% |
|---|---|
| Acrylsäure | 6,72 |
| Essigsäure | 0,22 |
| H₂O | 11,06 |
| Formaldehyd | 0,14 |
| Acrolein | 0,05 |
| Ameisensäure | 0,03 |
| Maleinsäureanhydrid | 0,06 |
| Benzoesäure | 0,01 |
| Propan | 14,62 |
| Propylen | 0,28 |
| Ethan | 1,02 |
| Ethylen | 0,18 |
| CO₂ | 2,03 |
| CO | 0,52 |
| N₂ | 59,86 |
| O₂ | 3,20 |
| Propionsäure | 0,0032 |

U^{A}_{C}, der Acroleinumsatz am Ende der Reaktionszone C, beträgt 68,1 mol.-%.

U^{A}_{D}, der Acroleinumsatz am Ende der Reaktionszone D, beträgt 99,3 mol.-%.

In beiden Reaktionsstufen durchströmt das Reaktionsgasgemisch die beiden Kontaktrohre von oben nach unten.

Die Gehaltsanalysen erfolgen mittels gaschromatographischer Analyse.

Die Acrylsäure wird aus dem Produktgasgemisch wie in den beispielhaften Ausführungen der DE-A 10 2004 032 129 abgetrennt und das Restgas als gasförmiger Ausgangsstrom 2 in die heterogen katalysierte Dehydrierung rückgeführt.

Das Verfahren kann auch wie beschrieben, jedoch mit dem Unterschied durchgeführt werden, dass in der Reaktionszone A jede Katalysatorhorde nur mit der gleichen Menge Dehydrierkatalysator beschickt ist, d. h., ohne Mitverwendung von Inertmaterial zu Verdünnungszwecken.

### IV. Änderung des Verfahrens gemäß III. mit zunehmender Betriebsdauer

Mit zunehmender Betriebdauer der Versuchsanlage sinkt der Propylengehalt im Produktgasgemischstrom A, selbst wenn der Dehydrierkatalysator regeneriert oder durch eine frisch produzierte Charge ersetzt wird.

Gleichzeitig ist eine Abnahme der maximalen Temperatur im ersten Katalysatorfestbett (in Strömungsrichtung) des Hordenreaktors feststellbar.

Mit Erzeugung des Treibstrahlgemischstroms in der Abfolge Restgas, Frischpropan, Wasserdampf (dann indirekter Wärmeaustausch) und dann erst molekularer Wasserstoff lassen sich die ursprünglichen Ergebnisse bei Neubetrieb wieder herstellen.

## Patentansprüche

1. Verfahren zur Herstellung von Acrolein, oder Acrylsäure oder deren Gemisch aus Propan, bei dem man
A) dem Einlass in eine erste Reaktionszone A einen Reaktionsgasgemischeingangsstrom A zuführt, der durch Zusammenführung von wenigstens vier voneinander verschiedenen gasförmigen Ausgangsströmen 1, 2, 3 und 4 mit der Maßgabe erzeugt wurde, dass die drei gasförmigen Ausgangsströme 1, 2 und 3 Propan enthalten, der gasförmige Ausgangsstrom 4 molekularer Wasserstoff und der gasförmige Ausgangsstrom 3 Frischpropan ist,
den Reaktionsgasgemischeingangsstrom A in der Reaktionszone A durch wenigstens ein Katalysatorbett führt, an welchem, gegebenenfalls unter Zufuhr weiterer Gasströme, durch partielle heterogen katalysierte Dehydrierung des Propan ein Propan und Propylen enthaltender Produktgasgemischstrom A gebildet wird,
den Produktgasgemischstrom A durch den Auslass aus der ersten Reaktionszone A aus selbiger herausführt und dabei in zwei Produktgasgemisch A-Teilströme 1 und 2 mit identischer Zusammensetzung aufteilt und den Produktgasgemisch A-Teilstrom 1 in erster Kreisgasfahrweise als gasförmigen Ausgangsstrom 1 in die erste Reaktionszone A rückführt,
den Produktgasgemisch A-Teilstrom 2 gegebenenfalls in eine erste Trennzone A führt, um in selbiger von in ihm enthaltenen, von Propan und Propylen verschiedenen Bestandteilen eine Teilmenge oder mehr abzutrennen, und **dadurch** einen verbleibenden, Propan und Propylen enthaltenden, Produktgasgemischstrom A' zu erzeugen,
B) den Produktgasgemischstrom A-Teilstrom 2 oder den Produktgasgemischstrom A' in einer zweiten Reaktionszone B zu Beschickung wenigstens eines Oxidationsreaktors verwendet und in dem wenigstens einen Oxidationsreaktor das im Produktgasgemisch A-Teilstrom 2 oder im Produktgasgemischstrom A' enthaltene Propylen einer selektiven heterogen katalysierten partiellen Gasphasenoxidation mit molekularem Sauerstoff zu einem Acrolein, oder Acrylsäure, oder deren Gemisch als Zielprodukt, nicht umgesetztes Propan und gegebenenfalls nicht umgesetztes Propylen sowie überschüssigen molekularen Sauerstoff enthaltenden Produktgasgemischstrom B unterwirft,
den Produktgasgemischstrom B aus der Reaktionszone B herausführt und in einer zweiten Trennzone B im Produktgasgemischstrom B enthaltenes Zielprodukt abtrennt und von dem dabei verbleibenden, nicht umgesetztes Propan, molekularen Sauerstoff sowie gegebenenfalls nicht umgesetztes Propylen enthaltenden Restgas wenigstens eine nicht umgesetztes Propan, molekularen Sauerstoff und gegebenenfalls nicht umgesetztes Propylen enthaltende Teilmenge in zweiter Kreisgasfahrweise als gasförmigen Ausgangsstrom 2 mit der Maßgabe in die Reaktionszone A rückführt, dass man die gasförmigen Ausgangsströme 2, 3 und 4 sowie gegebenenfalls zusätzliche vom gasförmigen Ausgangsstrom 1 verschiedene gasförmige Ausgangsströme zu einem gasförmigen Treibstrahlgemischstrom vereinigt und anschließend mit dem gasförmigen Treibstrahlgemischstrom als Treibstrahl eine Strahlpumpe, die eine Treibdüse, eine Mischstrecke, einen Diffusor und einen Saugstutzen umfasst, betreibt, wobei die Förderrichtung des durch die Treibdüse über die Mischstrecke und den Diffusor entspannten Treibstrahls in den Einlass der ersten Reaktionszone A sowie die Saugwirkung des Saugstutzens in Richtung des den Produktgasgemischstrom A führenden Auslass der ersten Reaktionszone A weist und dabei durch den im Saugstutzen erzeugten Unterdruck unter Aufteilung des Produktgasgemischstroms A in die beiden Teilströme 1 und 2 den Produktgasgemisch A-Teilstrom 1 ansaugt, bei gleichzeitiger Vermischung mit dem Treibstrahl durch die Mischstrecke über den Diffusor transportiert und den dabei gebildeten Reaktionsgasgemischeingangsstrom A in den Einlass der ersten Reaktionszone A entlässt,
**dadurch gekennzeichnet,**
**dass** man zunächst die gasförmigen Ausgangsströme 2 und 3 sowie gegebenenfalls zusätzliche, von den gasförmigen Ausgangsströmen 1 und 4 verschiedene gasförmige Ausgangsströme in beliebiger Abfolge zu einem gasförmigen Ausgangsgemischstrom vereinigt und erst dem gasförmigen Ausgangsgemischstrom unter Ausbildung des gasförmigen Treibstrahlgemischstroms den gasförmigen Ausgangsstrom 4 hinzufügt.

2. Verfahren nach Anspruch 1, **dadurch gekennzeichnet, dass** der Reaktionsgasgemischeingangsstrom A durch Zusammenführung von fünf voneinander verschiedenen gasförmigen Ausgangsströmen 1, 2, 3, 4 und 5 erzeugt wurde und der gasförmige Ausgangsstrom 5 Wasserdampf ist.

3. Verfahren nach Anspruch 2, **dadurch gekennzeichnet, dass** die Vereinigung der von den gasförmigen Ausgangsströmen 1 und 4 verschiedenen gasförmigen Ausgangsströme zum gasförmigen Ausgangsgemischstrom in der Abfolge gasförmiger Ausgangsstrom 2, gasförmiger Ausgangsstrom 5 und dann gasförmiger Ausgangsstrom 3 erfolgt.

4. Verfahren nach einem der Ansprüche 1 bis 3, **dadurch gekennzeichnet, dass** vom Zeitpunkt der Ausbildung des Treibstrahlgemischstroms bis zu dem Zeitpunkt, an dem der Reaktionsgasgemischeingangsstrom A das in Strömungsrichtung erste Katalysatorbett der Reaktionszone A erreicht, nicht mehr als 30 Sekunden vergehen.

5. Verfahren nach einem der Ansprüche 1 bis 3, **dadurch gekennzeichnet, dass** vom Zeitpunkt der Ausbildung des Treibstrahlgemischstroms bis zu dem Zeitpunkt, an dem der Reaktionsgasgemischeingangsstrom A das in Strömungsrichtung erste Katalysatorbett der Reaktionszone A erreicht, nicht mehr als 10 Sekunden vergehen.

6. Verfahren nach einem der Ansprüche 1 bis 5, **dadurch gekennzeichnet, dass** die Reaktionszone A ein Hordenreaktor ist.

7. Verfahren nach einem der Ansprüche 1 bis 6, **dadurch gekennzeichnet, dass** die partielle heterogen katalysierte Dehydrierung des Propan in der Reaktionszone A autotherm erfolgt.

## Claims

1. A process for preparing acrolein or acrylic acid or a mixture thereof from propane, in which
A) a reaction gas mixture input stream A which has been obtained by combining at least four different gaseous starting streams 1, 2, 3 and 4 with the proviso that the three gaseous starting streams 1, 2 and 3 comprise propane, gaseous starting stream 4 is molecular hydrogen and gaseous starting stream 3 is fresh propane is fed to the inlet into a first reaction zone A,
reaction gas mixture input stream A is conducted in reaction zone A through at least one catalyst bed over which, if appropriate with supply of further gas streams, a product gas mixture stream A comprising propane and propylene is formed by partial heterogeneously catalyzed dehydrogenation of propane,
product gas mixture stream A is conducted out of the first reaction zone A by discharge therefrom and divided into two product gas mixture A substreams 1 and 2 with identical composition, and product gas mixture A substream 1, in a first cycle gas method, is recycled into the first reaction zone A as the gaseous starting stream 1,
product gas mixture A substream 2 is, if appropriate, conducted into a first separating zone A in order to remove therefrom a portion or more of the constituents other than propane and propylene present therein to generate a remaining product gas mixture stream A' comprising propane and propylene,
B) product gas mixture stream A substream 2 or product gas mixture stream A' are used in a second reaction zone B to charge at least one oxidation reactor and, in the at least one oxidation reactor, the propylene present in product gas mixture A substream 2 or in product gas mixture stream A' is subjected to a selective heterogeneously catalyzed partial gas phase oxidation with molecular oxygen to give a product gas mixture stream B comprising acrolein or acrylic acid or a mixture thereof as the target product, unconverted propane and, if appropriate, unconverted propylene, and also excess molecular oxygen,
product gas mixture stream B is conducted out of reaction zone B and, in a second separating zone B, target product present in product gas mixture stream B is removed and, from the remaining residual gas comprising unconverted propane, molecular oxygen and any unconverted propylene, at least a portion comprising unconverted propane, molecular oxygen and any unconverted propylene, in a second cycle gas method, is recycled into reaction zone A as the gaseous starting stream 2 with the proviso that gaseous starting streams 2, 3 and 4 and any additional gaseous starting streams other than gaseous starting stream 1 are combined to give a gaseous motive jet mixture stream, and a jet pump which comprises a motive nozzle, a mixing zone, a diffuser and a suction nozzle is subsequently operated with the gaseous motive jet mixture stream as the motive jet, the conveying direction of the motive jet decompressed through the motive nozzle via the mixing zone and the diffuser pointing into the inlet of the first reaction zone A and the sucking action of the suction nozzle in the direction of the outlet conducting product gas mixture stream A of the first reaction zone A, and the reduced pressure generated in the suction nozzle, with division of product gas mixture stream A into the two substreams 1 and 2, sucks in product gas mixture A substream 1 and transports it through the mixing zone via the diffuser with simultaneous mixing with the motive jet, and the reaction gas mixture input stream A formed in this way is released into the inlet of the first reaction zone A,
wherein
gaseous starting streams 2 and 3 and any additional gaseous starting streams other than gaseous streams 1 and 4 are first combined in any sequence to give a gaseous starting mixture stream and gaseous starting stream 4 is only then added to the gaseous starting mixture stream to form the gaseous motive jet mixture stream.

2. The process according to claim 1, wherein reaction gas mixture input stream A has been obtained by combining five different gaseous starting streams 1, 2, 3, 4 and 5 and gaseous starting stream 5 is steam.

3. The process according to claim 2, wherein the gaseous starting streams other than gaseous starting streams 1 and 4 are combined to give the gaseous starting mixture stream in the sequence of gaseous starting stream 2, gaseous starting stream 5 and then gaseous starting stream 3.

4. The process according to any of claims 1 to 3, wherein not more than 30 seconds elapse from the time of formation of the motive jet mixture stream to the time at which reaction gas mixture input stream A reaches the first catalyst bed of reaction zone A in flow direction.

5. The process according to any of claims 1 to 3, wherein not more than 10 seconds elapse from the time of formation of the motive jet mixture stream to the time at which reaction gas mixture input stream A reaches the first catalyst bed of reaction zone A in flow direction.

6. The process according to any of claims 1 to 5, wherein reaction zone A is a tray reactor.

7. The process according to any of claims 1 to 6, wherein the partial heterogeneously catalyzed dehydrogenation of propane is effected autothermally in reaction zone A.

## Revendications

1. Procédé de fabrication d'acroléine ou d'acide acrylique ou de leur mélange à partir de propane, selon lequel
A) un courant de mélange gazeux réactionnel d'entrée A, qui a été généré par combinaison d'au moins quatre courants gazeux initiaux différents les uns des autres 1, 2, 3 et 4, est introduit dans l'entrée d'une première zone de réaction A, à condition que les trois courants gazeux initiaux 1, 2 et 3 contiennent du propane, que le courant gazeux initial 4 soit de l'hydrogène moléculaire et que le courant gazeux initial 3 soit du propane frais,
le courant de mélange gazeux réactionnel d'entrée A traverse au moins un lit catalytique dans la zone de réaction A, sur lequel, éventuellement avec introduction de courants gazeux supplémentaires, un courant de mélange gazeux de produits A contenant du propane et du propylène est formé par déshydrogénation partielle sous catalyse hétérogène du propane,
le courant de mélange gazeux de produits A est déchargé de la première zone de réaction A par la sortie de celle-ci et divisé en deux courants partiels 1 et 2 du mélange gazeux de produits A de composition identique, et le courant partiel 1 du mélange gazeux de produits A est recyclé dans la première zone de réaction A en tant que courant gazeux initial 1 selon un premier mode de circulation de gaz,
le courant partiel 2 du mélange gazeux de produits A est éventuellement conduit dans une première zone de séparation A, afin d'y séparer une partie ou plus des constituants différents du propane et du propylène qu'il contient, et de générer ainsi un courant de mélange gazeux de produits A' restant, contenant du propane et du propylène,
B) le courant partiel 2 du courant de mélange gazeux de produits A ou le courant de mélange gazeux de produits A' est utilisé dans une seconde zone de réaction B pour l'alimentation d'au moins un réacteur d'oxydation et, dans le ou les réacteurs d'oxydation, le propylène contenu dans le courant partiel 2 du mélange gazeux de produits A ou dans le courant de mélange gazeux de produits A' est soumis à une oxydation partielle sélective en phase gazeuse sous catalyse hétérogène avec de l'oxygène moléculaire pour former un courant de mélange gazeux de produits B contenant de l'acroléine ou de l'acide acrylique ou leur mélange en tant que produit cible, du propane non réagi et éventuellement du propylène non réagi, ainsi que de l'oxygène moléculaire en excès,
le courant de mélange gazeux de produits B est déchargé de la zone de réaction B et le produit cible contenu dans le courant de mélange gazeux de produits B est séparé dans une seconde zone de séparation B, et, à partir du gaz résiduel restant contenant du propane non réagi, de l'oxygène moléculaire et éventuellement du propylène non réagi, au moins une partie contenant du propane non réagi, de l'oxygène moléculaire et éventuellement du propylène non réagi est recyclée dans la zone de réaction A en tant que courant gazeux initial 2 selon un second mode de circulation de gaz, à condition que les courants gazeux initiaux 2, 3 et 4, ainsi qu'éventuellement des courants gazeux initiaux différents du courant gazeux initial 1 supplémentaires soient combinés pour former un courant de mélange gazeux de jet d'entraînement, puis une pompe à jet, qui comprend une buse d'entraînement, une section de mélange, un diffuseur et une tubulure d'aspiration, est actionnée avec le courant de mélange gazeux de jet d'entraînement en tant que jet d'entraînement, le jet d'entraînement détendu par la buse d'entraînement via la section de mélange et le diffuseur étant dirigé vers l'entrée de la première zone de réaction A et l'action d'aspiration de la tubulure d'aspiration étant dirigée dans la direction de la sortie de la première zone de réaction A qui conduit le courant de mélange gazeux de produits A, et la sous-pression générée dans la tubulure d'aspiration permettant ainsi d'aspirer le courant partiel 1 du mélange gazeux de produits A, avec division du courant de mélange gazeux de produits A en deux courants partiels 1 et 2, de le transporter par le diffuseur avec un mélange simultané avec le jet d'entraînement via la section de mélange, et d'éjecter le courant de mélange gazeux réactionnel d'entrée A ainsi formé dans l'entrée de la première zone de réaction A,
**caractérisé en ce que,**
les courants gazeux initiaux 2 et 3, ainsi qu'éventuellement des courants gazeux initiaux différents des courant gazeux initiaux 1 et 4 supplémentaires sont tout d'abord combinés dans un ordre quelconque pour former un courant de mélange gazeux initial puis, seulement alors, le courant gazeux initial 4 est ajouté au courant de mélange gazeux initial pour former le courant de mélange gazeux de jet d'entraînement.

2. Procédé selon la revendication 1, **caractérisé en ce que** le courant de mélange gazeux réactionnel d'entrée A est généré par combinaison de cinq courants gazeux initiaux différents les uns des autres 1, 2, 3, 4 et 5 et **en ce que** le courant gazeux initial 5 est de la vapeur d'eau.

3. Procédé selon la revendication 2, **caractérisé en ce que** la combinaison des courants gazeux initiaux différents des courants gazeux initiaux 1 et 4 pour former le courant de mélange gazeux initial a lieu dans l'ordre courant gazeux initial 2, courant gazeux initial 5, puis courant gazeux initial 3.

4. Procédé selon l'une quelconque des revendications 1 à 3, **caractérisé en ce qu'**une durée inférieure ou égale à 30 secondes s'écoule entre le moment de la formation du courant de mélange de jet d'entraînement et le moment auquel le courant de mélange gazeux réactionnel d'entrée A atteint le premier lit catalytique de la zone de réaction A dans la direction de l'écoulement.

5. Procédé selon l'une quelconque des revendications 1 à 3, **caractérisé en ce qu'**une durée inférieure ou égale à 10 secondes s'écoule entre le moment de la formation du courant de mélange de jet d'entraînement et le moment auquel le courant de mélange gazeux réactionnel d'entrée A atteint le premier lit catalytique de la zone de réaction A dans la direction de l'écoulement.

6. Procédé selon l'une quelconque des revendications 1 à 5, **caractérisé en ce que** la zone de réaction A est un réacteur à étages.

7. Procédé selon l'une quelconque des revendications 1 à 6, **caractérisé en ce que** la déshydrogénation partielle sous catalyse hétérogène du propane dans la zone de réaction A a lieu de manière autotherme.
